# EUROPEAN PATENT SPECIFICATION

(11) **EP 1 476 197 B1**
(45) Date of publication and mention of the grant of the patent: **07.01.2009**
(21) Application number: 03704133.2
(22) Date of filing: 24.02.2003
(51) Int. Cl.: A61K 47/48, A61K 39/02

(54) **SYNTHESIS OF LIPOPOLYSACCHARIDE-PROTEIN CONJUGATE VACCINES VIA THE LIPID A REGION FOLLOWING REMOVAL OF THE GLYCOSIDIC PHOSPHATE RESIDUE**
SYNTHESE VON LIPOPOLYSACCHARID-PROTEIN KONJUGATVACCINEN ÜBER DIE LIPID-A REGION NACH ENTFERNUNG DES GLYCOSIDISCHEN PHOSPHATRESIDÜS
SYNTHESE DE VACCINS CONJUGUES DE PROTEINE-LIPOPOLYSACCHARIDE AU MOYEN DE LA REGION LIPIDIQUE APRES RETRAIT DU RESIDU DE PHOSPHATE GLYCOSIDIQUE

(30) Priority: 22.02.2002 US 358384 P
(43) Date of publication of application: 17.11.2004
(73) Proprietor: NATIONAL RESEARCH COUNCIL OF CANADA, Ottawa, Ontario K1A OR6 (CA)
(72) Inventor: JENNINGS, Harold, Gloucester, Ontario K1J 6G6 (CA); MIESZALA, Malgorzata, 51-507 Wroclaw (PL); KOGAN, Grigorij, 851 05 Bratislava (SK); ZOU, Wei, Ottawa, Ontario K1V 1P4 (CA); RICHARDS, James, C., Ottawa, Ontario K1M 0Z3 (CA); COX, Andrew, Gloucester, Ontario K1J 6K4 (CA); MOXON, Richard, Oxford OX2 7AX (GB)
(74) Representative: Smaggasgale, Gillian Helen
(86) International application number: PCT/CA2003/000254
(87) International publication number: WO 2003/070282

(56) References cited:
- WO-A-01/78787
- MUELLER-LOENNIES, SVEN ET AL: "A novel strategy for the synthesis of neoglycoconjugates from deacylated deep rough lipopolysaccharides" JOURNAL OF ENDOTOXIN RESEARCH (2002), 8(4), 295-305 , XP008022939
- MIESZALA M ET AL: "Conjugation of meningococcal lipooligosaccharides through their lipid A terminus conserves their inner epitopes and results in conjugate vaccines having improved immunological properties" CARBOHYDRATE RESEARCH, ELSEVIER SCIENTIFIC PUBLISHING COMPANY. AMSTERDAM, NL, vol. 338, no. 2, 20 January 2003 (2003-01-20), pages 167-175, XP004401753 ISSN: 0008-6215
- RAETZ C R H: "BIOCHEMISTRY OF ENDOTOXINS" ANNUAL REVIEW OF BIOCHEMISTRY, PALTO ALTO, CA, US, vol. 59, 1990, pages 129-170, XP001024252 ISSN: 0066-4154
- VERHEUL A F M ET AL: "PREPARATION, CHARACTERIZATION, AND IMMUNOGENICITY OF MENINGOCOCCAL IMMUNOTYPE L2 AND L3,7,9 PHOSPHOETHANOLAMINE GROUP-CONTAINING OLIGOSACCHARIDE-PROTEIN CONJUGATES" INFECTION AND IMMUNITY, AMERICAN SOCIETY FOR MICROBIOLOGY. WASHINGTON, US, vol. 59, no. 3, 1 March 1991 (1991-03-01), pages 843-851, XP002032436 ISSN: 0019-9567 cited in the application

## Description

### FIELD OF THE INVENTION

This invention relates to lipopolysaccharide-protein conjugate vaccines with optimum presentation of oligosaccharide epitopes that have improved immunogenic properties following coupling to protein carriers via the lipid A region.

### BACKGROUND OF THE INVENTION

Despite the phenomenal success of polysaccharide-protein conjugate vaccines in combating serious bacterial infections caused by encapsulated bacteria, this technology is not applicable to some important bacterial pathogens. This is due to the fact that some pathogenic bacteria are not encapsulated. Non-typable *Haemophilus influenzae,* a major causative agent of respiratory and *otitis media* infections in infants, is in this category. Additionally some bacteria have capsular polysaccharides, which even when conjugated, are poorly immunogenic. The α2-8 polysialic acid capsule of group B *Neisseria meningitidis,* which is responsible for half of all cases of meningococcal meningitis in the western world, is a prime example of this. Gram-negative bacteria have an outer membrane comprised of components including proteins, lipoproteins, phospholipids, and glycolipids. The glycolipids comprise primarily endotoxin lipopolysaccharides (LPS). LPS are molecules comprised of a) a Lipid A portion which consists of a glucosamine disaccharide that is substituted with phosphate groups and long chain fatty acids in ester and amide linkages; b) a core polysaccharide which is attached to Lipid A by an eight carbon sugar, Kdo (2-keto-3-deoxyoctulosonic acid), and generally contains heptose, glucose, galactose, and N-acetylglucosamine; and, optionally, c) *O*-specific side chains comprised of repeating oligosaccharide units which, depending on the genera and species of bacteria, may contain mannose, galactose, D-glucose, N-acetylgalactosamine, N-acetylglucosamine, L-rhamnose, or dideoxyhexose (abequose, colitose, tyvelose, paratose, trehalose). LPS which lacks repeating O-side chains is sometimes referred to as short chain lipopolysaccharide, or as lipooligosaccharide (or LOS). In this application, the terms lipopolysaccharide (or LPS) and lipooligosaccharide (or LOS) may be used interchangeably.

The major antigenic determinants of Gram-negative bacteria are believed to reside in the complex carbohydrate structure of LPS. These carbohydrate structures vary significantly, even among different species of the same genus of Gram-negative bacteria, primarily because of variations in one or more of the sugar composition, the sequence of oligosaccharides, the linkage between the monomeric units of the oligosaccharides, between the oligosaccharides themselves, and due to substitutions/modifications of the oligosaccharides.

LPS is a bacterial component which has potential as a vaccine immunogen because of the antigenic determinants ("epitopes") residing in its carbohydrate structures. However, the chemical nature of LPS detracts from its use in vaccine formulations; i.e., active immunization with LPS is unacceptable due to the inherent toxicity, in some animals, of the Lipid A portion. The pathophysiological effects induced (directly or indirectly) by Lipid A of LPS in the bloodstream include fever, leucopenia, leucocytosis, the Shwartzman reaction, disseminated intravascular coagulation, abortion, and in larger doses, shock and death.

In the absence of suitable capsular polysaccharide components with which to synthesize conjugate vaccines, the use of alternative carbohydrate antigens as components of conjugate vaccines is being actively explored. One of these antigens is the lipopolysaccharide (LPS) or lipooligosaccharide (LOS) .The LPS of *N. meningitidis* and non-typable *H. influenzae* are in this latter category (i.e., LOS) and both have been implicated in the immune response to natural infections caused by their respective organisms. Unfortunately the LOS of both organisms are extremely toxic and cannot be used as vaccines either alone or as conjugates. However, the toxicity of LOS resides only in its lipid A moiety, and strategies have been devised to surmount this problem. In the first conjugate vaccines this was achieved by prior removal of the lipid A moiety from the LOS by mild acid hydrolysis. The resultant non-toxic truncated LOS was then conjugated either directly or by using a spacer molecule to a protein through the terminal 2-keto-3-deoxyoctulosonic acid (KDO) residue. In a more recent study the intact LOS of non-typable *H. influenzae* was detoxified using anhydrous hydrazine prior to conjugation. This reagent O-deacylates the lipid A moiety which results in detoxification of the LOS. Conjugation of the detoxified LOS was accomplished by linking the carboxylate functions of an internal KDO residue to a protein by means of an adipic acid dihydrazide spacer. While conjugates made with acid hydrolyzed LOS have been demonstrated to produce antibodies that react with their respective native LOS, some of which even exhibit protective properties in animals, their immune response may not be optimal. This is because the point of cleavage at the KDO residue is within the area of internal epitopes (i.e., inner core) of the LOS, and in consequence the structure of these epitopes may be impaired. Inner core internal epitopes have been shown to be highly conserved across strains of a specific species, and as such will be attractive vaccine candidates. Additionally, inner core oligosaccharides are a sensible choice because of the fact that structural similarity between the outer core oligosaccharides of the LOS and mammalian tissue antigens could lead to a poor immunogenic host response or autoimmune diseases.

There is, therefore, a continuing requirement for vaccines of increased efficacy and reduced side effects. Many approaches have been taken over the years. An approach, outlined above, which has been popular recently is partial deacylation of lipopolysaccharide moieties of infective microorganism, deacylation being used to remove esterified fatty acid side chains of lipid A, the esterified fatty acid side chains being major contributors to the toxicity of lipid A. Gu et al in US Patent 6,207,157 and its Divisional Patent Application Publication US 2002/0001389A1 disclose a conjugate vaccine for non-typable *Haemophilus influenzae.* This comprises a lipooligosaccharide from which esterified fatty acid side chains have been removed from lipid A to form a detoxified LOS (dLOS) which is then linked covalently to an immunogenic carrier. The ester-linked fatty acids are removed using hydrazine prior to conjugation to the linker adipic acid dihydrazide (ADH) prior to conjugation to an immunogenic carrier protein. Gu et al in Vaccine 3463, 1-8 (2002) teach the production of a lipooligosaccharide-based conjugate vaccine against non-typable *Haemophilus influenzae.* They linked adipic acid dehydrazide reacted lipooligosaccharide (AH-dLOS) to tetanus toxoid.

Another approach has been that of Plested at al in WO01/22994 who disclose a vaccine for treatment of disease caused by *Neisseria meningitidis* based on elements of the inner core lipopolysaccharide. A point which appears to be critical in their approach is the presence of conserved internal epitopes that are present in a majority of disease causing isolates.

A further approach has been that of Richards et al in WO02/16440 who disclose a conserved triheptosyl inner core moiety of a lipopolysaccharide substantially free of variable outer core oligosaccharide chain extension and its use in vaccines for preventing *Haemophilus influenzae* infections.

In Infection and Immunity, Mar. 1991, p 843-851, Verheul et al disclosed a method for the well-defined coupling of phosphoethanolamine group (PEA)-and carboxylic acid group-containing polysaccharides and oligosaccharides to proteins without the need for extensive modification of the carbohydrate antigens. The carboxylic acid group of the terminal 2-keto-3-deoxyoctulosonic acid moiety was utilized to introduce a thiol function in meningococcal immunotype L2 and L3,7,9 lipopolysaccharide-derived oligosaccharides. The thiol group-containing oligosaccharides were subsequently coupled to bromoacetylated proteins. Immunotype L2 and L3,7,9 PEA group-containing oligosaccharide tetanus toxoid conjugates were prepared, and their immunogenicities were studied in rabbits. Both the immunotype L2 and immunotype L3,7,9 conjugates evoked high immunoglobulin G (IgG) antibody titers after the first booster injection. These conjugates also displayed an ability to induce long-lasting IgG antibody levels which could be detected until 9 months after one booster injection at week 3. The adjuvant Quil A enhanced the immune response to all the conjugates to a minor extent, which is in contrast with reported adjuvant effects of Quil A on these types of antigens in mice. A conjugate prepared from the dephosphorylated L3,7,9 oligosaccharides evoked a significantly lower IgG response than a similar PEA-containing conjugate, and enzyme-linked immunosorbent assay inhibition studies indicated adifferent epitope specificity. Furthermore, antisera elicited with the complete bacteria contained antibodies directed against PEA-containing epitopes, which stresses the importance of the presence of unmodified PEA groups in meningococcal lipopolysaccharide derived oligosaccharide-protein conjugates. The procedure developed offers an elegant solution for the specific coupling of meningococcal PEA-containing oligosaccharides to proteins and may therefore be a very useful tool in the development of a vaccine against group B meningococci. In Infection and Immunity, Jan. 1984, p 407-412, Jennings et al disclosed a method in which oligosaccharides were obtained by the mild acid hydrolysis of the lipopolysaccharides from a number of different strains of Neisseria meningitidis, serotypes L2, L3, L4, L5, and L10. The dephosphorylated oligosaccharides were conjugated to tetanus toxoid as their 2-(4-isothiocyanatophenyl)-ethylamine derivatives, which resulted in the incorporation of from 18 to 38 oligosaccharides per molecule of tetanus toxoid. When injected in rabbits, the conjugates produced oligosaccharide-specific antibodies which were predominantly serologically specific but which also exhibited some cross-reactivity. These serological results can be attributed to regions of structural dissimilarity and similarity within the oligosaccharides. The oligosaccharide-specific antibodies were also lipopolysaccharide serotype specific, thus indicating that the oligosaccharides are the determinants associated with this serotype specificity. Consistent with the serological results, the conjugate antisera were bactericidal for the homologous serotype meningococcal organisms and in some cases for heterologous serotype organisms.

In our studies we have found that one factor, which does not appear to have been fully appreciated is the importance of coupling the carbohydrate to protein carrier appropriately, in such a way that crucial antigenic and potentially immunogenic epitopes would not be modified or compromised by the conjugation strategy employed. We became particularly interested in removing phosphate, pyrophosphate or pyrophosphorylethanolamine groups from the reducing end of the lipid A region of the LOS molecule thereby creating an active hemicaetal group through which the LOS can be linked to protein carriers. The fact that all known LOS and LPS contain similar terminal phosphorylated substituents in the lipid A region of the molecules means that this procedure may be important in the synthesis of conjugate vaccines because it would have wide applicability. The basic conceptual approach is, with hindsight, surprisingly simple. Instead of using a partial epitope (e.g. liberated core oligosaccharide) and linking this to a carrier, or using a deacylated moiety (which is detoxified) but is linked to the carrier in a way that does not preserve the integrity of conserved inner core oligosaccharide epitope, we ensure that the deacylated (detoxified) lipopolysaccharide is linked to the protein at a site remote from the internal epitopes through partial or complete dephosphorylation of the detoxified lipid A moiety and thereby preserve the integrity of said epitopes. Conveniently this is achieved with an alkaline phosphatase but any suitable means to this end will do. The resulting immunogens show remarkable promise. Utilizing immunogens consisting of conserved inner core oligosaccharide epitopes displayed on deacylated LPS conjugated to protein carriers via the lipid A terminus we have achieved some remarkable and surprisingly rewarding results.

### SUMMARY OF THE INVENTION

In a first aspect, the invention relates to an antigenic detoxified reducing Gram-negative bacterial lipooligosaccharide having a detoxified lipid A region and linkable to an immunologically acceptable carrier through the detoxified lipid A region, said antigenic detoxified reducing bacterial lipooligosaccharide consisting of a moiety of the formula: 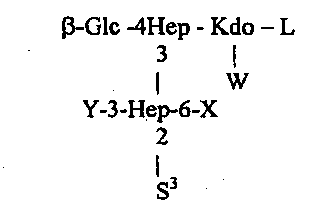
wherein L is the detoxified lipid A region;
Kdo is a 2-keto-3-deoxyoctulosonic acid moiety;
W is a 2-keto-3-deoxyoctulosonic acid moiety, a phosphate group, or a pyrophosphoethanolamine group;
Glc is glucose;
Hep is L-glycero-α-D-manno-heptose;
Y is phosphoethanolamine or hydrogen;
X is phosphoethanolamine when Y is hydrogen and X is hydrogen when Y is phosphoethanolamine; and
S³ is N-acetyl-α-glucosamine or L-glycero-α-D-manno-heptose.

The invention further relates to a conjugate vaccine for combating Gram-negative bacterium comprising an antigenic, detoxified lipopolysaccharide of the above first aspect linked to an immunologically acceptable carrier through the O-deacylated lipid A region.

The invention also relates to a conjugate vaccine for combating a Gram-negative bacterium comprising an antigenic, detoxified reducing bacterial lipooligosaccharide according to the above first aspect linked via a linker to an immunologically acceptable carrier through the O-deacylated A region.

The invention also relates to pharmaceutical compositions comprising the conjugate vaccine of the invention in association with an adjuvant. The pharmaceutical compositions of the invention may also comprise such as an adjuvant selected from the group consisting of Freund's adjuvant and Ribi. Although these adjuvant are not approved for use in humans, the skilled artisan will appreciate that other well known standard adjuvants may be used in the invention, including aluminum compounds (i.e. alum), chemically-modified lipopolysaccharide, suspensions of killed Bordetella pertussis, N-acetylmuramyl-L-alanyl-D-glutamine and other adjuvants known to one of ordinary skill in the art.

Another embodiment of the invention is the use of an antigenic, detoxified reducing bacterial lipooligosaccharide of the invention for combating a Gram-negative infection in a mammal.

A still further embodiment of the invention is the use of an antigenic, detoxified reducing bacterial lipooligosaccharide according to the first aspect of the invention in the manufacture of a medicament for combating a Gram-negative infection in a mammal. Preferably, the mammal is a human. In use, the route of administration may be intramuscular, subcutaneous, intraperitoneal, intraarterial, intravenous or intranasal; most preferably, the administering step is intramuscular. In use, the effective dose may be between about 10µg and about 50µg. Use may further comprise injecting between about 10µg and about 25µg at about 2 months and at about 13 months after the administering step. Alternatively, the use may further comprise injecting between about 10µg and about 25µg at about 2, 4 and 16 months after the administering step.

Although the use of hydrazine for detoxification of LPS or LOS is described herein, the use of any reagent or enzyme capable of removing ester-linked fatty acids from lipid A or achieving this through genetic modification or genetic engineering techniques is within the scope of the present invention. Dried LPS or LOS is suspended in liquid anhydrous hydrazine at a temperature of between 1°C and 100°C; preferably between 25°C and 75°C; more preferably, about 37°C for a period between 1 hour and 24 hours, most preferably for a period of about 2-3 hours. After removal of ester-linked fatty acids, the terminal glycose (e.g., glucosamine) residue in the lipid A region of dLOS (also referred to as LOS-OH or LPS-OH) is dephosphorylated. The use of any chemical reagent or enzyme capable of removing the phosphate or phosphate substituents from the terminal glycose of the LOS or LPS, or through genetic modification engineering techniques is within the scope of the invention. A preferred embodiment of the invention is to employ an enzyme, more preferably an enzyme of the group of alkaline phosphatase enzymes to effect removal of the terminal glycose phosphate of dLOS to give the dephosphorylated analogue, dLOS, de P (also referred to as LOS-OH, de P or LPS-OH, de P). The use of any linker or spacer capable of stably and efficiently conjugating dLOS to an immunogenic carrier protein is also contemplated. The use of linkers is well known in the conjugate vaccine field (see Dick et al., Conjugate Vaccines, J. M. Cruse and R. E. Lewis, Jr., eds., Karger, New York, pp. 48-114.

dLOS, de P may be directly covalently bonded to the carrier. This may be accomplished, for example, by reductive amination as described herein. In an alternative embodiment, dLOS, de P and the carrier are separated by a linker. In some instances, the presence of a spacer or linker may promote improved immunogenicity of the conjugate and more efficient coupling of the dLOS, de P with the carrier. Linkers separate antigenic dLOS, de P and the carrier and, when the carrier is also an antigen, the two antigenic components by chains whose length and flexibility can be adjusted as desired. Between the bifunctional sites, the chains can contain a variety of structural features, including heteroatoms and cleavage sites. Linkers also permit corresponding increases in translational and rotational characteristics of the antigens, increasing access of the binding sites to soluble antibodies. Suitable linkers include, for example, linkers such as adipic acid dihydrazide (ADH), ε-aminohexanoic acid, chlorohexanol dimethyl acetal, D-glucuronolactone and p-nitrophenyl amine. Coupling reagents contemplated for use in the present invention include hydroxysuccinimides and carbodiimides. Many other linkers and coupling reagents known to those of ordinary skill in the art are also suitable for use in the invention. Dick et al. discuss such compounds in detail, supra.

The presence of a carrier can increase the immunogenicity of the polysaccharide and/or oligosaccharide of conjugated LPS or LOS. In addition, antibodies raised against the carrier are medically beneficial. Polymeric immunogenic carriers can be a natural or synthetic material containing a primary and/or secondary amino group, an azido group or a carboxyl group. The carrier may be water soluble or insoluble.

Any one of a variety of immunogenic carrier proteins may be used in the conjugate vaccine of the present invention. Such classes of proteins include pili, outer membrane proteins and excreted toxins of pathogenic bacteria, nontoxic or "toxoid" forms of such excreted toxins, nontoxic proteins antigenically similar to bacterial toxins (cross-reacting materials or CRMs) and other proteins. Nonlimiting examples of bacterial toxoids contemplated for use in the present invention include tetanus toxin/toxoid, diphtheria toxin/toxoid, detoxified P. aeruginosa toxin A, cholera toxin/toxoid, pertussis toxin/toxoid and Clostridium perfringens exotoxins/toxoid. The toxoid forms of these bacterial toxins are preferred. The use of viral proteins (i.e. hepatitis B surface/core antigens; rotavirus VP 7 protein and respiratory syncytial virus F and G proteins) is also contemplated.

CRMs include CRM₁₉₇, antigenically equivalent to diphtheria toxin (Pappenheimer et al., Immunochem., 9:891-906, 1972) and CRM3201, a genetically manipulated variant of pertussis toxin (Black et al., Science, 240:656-659, 1988). The use of immunogenic carrier proteins from non-mammalian sources including keyhole limpet hemocyanin, horseshoe crab hemocyanin and plant edestin is also within the scope of the invention.

There are many coupling methods which can be envisioned for dLOS-protein conjugates. Applicant teaches in the examples set forth below, dLOS is selectively activated by complete removal of phosphate groups from the reducing terminus glucosamine with an alkaline phosphatase followed by sodium cyanoborohydride -mediated reductive amination of dLOS, de P to epsilon amino groups of exposed lysine residues on TT or CRM. Alternatively, another method for producing the conjugates containing a linker involves cystamine derivatization of dLOS, by, for example, reductive amination, followed by generation of a thio-group and disulfide conjugation to N-bromoacetylated-TT. Other methods well known in the art for effecting conjugation of oligosaccharides which preserve the integrity of internal epitopes to immunogenic carrier proteins are also within the scope of the invention.

For effective introduction of a linker, the molar ratio of linker to dLOS in the reaction mixture is typically between about 10:1 and about 250:1. A molar excess of linker is used to ensure more efficient coupling and to limit dLOS-dLOS coupling. In a preferred embodiment, the molar ratio is between about 50:1 and about 150:1; in a most preferred embodiment, the molar ratio is about 100:1. Similar ratios of linker-dLOS to TT in the reaction mixture are contemplated. In a preferred embodiment, in the final dLOS-carrier protein conjugate, the molar ratio of dLOS, de P to carrier is between about 15 and about 75, preferably between about 25 and about 50.

Immunogenicity of the conjugates in both mice and rabbits is enhanced by the use of monophosphoryl lipid A plus trehalose dimycolate (Ribi-700; Ribi Immunochemical Research, Hamilton, Mont.) as an adjuvant. Although this adjuvant is not approved for use in humans, the skilled artisan will appreciate that other well known standard adjuvants may be used in the invention, including aluminum compounds (i.e. alum), chemically-modified lipopolysaccharide, suspensions of killed Bordetella pertussis, N-acetylmuramyl-L-alanyl-D-glutamine and other adjuvants known to one of ordinary skill in the art. The use of aluminum compounds is particularly preferred. Such adjuvants are described by Warren et al. (Ann. Rev. Bioche., 4:369-388, 1986).

The dLOS-carrier protein conjugates for parenteral administration may be in the form of a sterile injectable preparation, such as a sterile injectable aqueous or oleaginous suspension. This suspension may be formulated according to methods well known in the art using suitable dispersing or wetting agents and suspending agents. The sterile injectable preparation may also be a sterile injectable solution or suspension in a parenterally acceptable diluent or solvent, such as a solution in 1,3-butanediol. Suitable diluents include, for example, water, Ringer's solution and isotonic sodium chloride solution. In addition, sterile fixed oils may be employed conventionally as a solvent or suspending medium. For this purpose, any bland fixed oil may be employed including synthetic mono- or diglycerides. In addition, fatty acids such as oleic acid may likewise be used in the preparation of injectable preparations.

The conjugate vaccine of the invention may be in soluble or microparticular form, or may be incorporated into microspheres or microvesicles, including liposomes. Although various routes of vaccine administration including, for example, intramuscular, subcutaneous, intraperitoneal and intraarterial are contemplated, the preferred route is intramuscular administration. In a preferred embodiment, the dosage of the conjugate administered will range from about 10µg to about 50µg of conjugated carbohydrate. In a more preferred embodiment, the amount administered will be between about 20µg and about 40µg. In a most preferred embodiment, the amount administered is about 25µg. Greater doses may be administered on the basis of body weight. The exact dosage can be determined by routine dose/response protocols known to one of ordinary skill in the art.

The vaccine of the invention may be administered to warm-blooded mammals of any age and are adapted to induce active immunization in young mammals, particularly humans. As a childhood vaccine, the conjugate is administered at about 2 to 4 months of age. Typically, two booster injections of between about 10µg and about 25µg conjugated carbohydrates are administered at about 2 and again about 13 months after the initial injection. Alternatively, three booster injections are given at 2, 4 and 16 months after the initial injection.

The IgG antibodies elicited by systemic administration of the conjugate vaccine will transfer to local mucosa and inactivate bacterial inoculum on mucosal surfaces (i.e., nasal passages). Secretory IgA will also play a role in mucosal immunity if the conjugate vaccine is administered to the mucosa (i.e. intranasally). Thus, the conjugate vaccine will prevent local, as well as systemic, bacterial infection.

### BRIEF DESCRIPTION OF THE DRAWINGS

Figure 1 illustrates structures of the truncated (L7-OS) and full length (L7-OH, de P) oligosaccharides prior to conjugation.
Figure 2 illustrates comparative ELISA titers of individual mouse serum following immunization with either L7-OS-TT or L7-OH, de P-TT (1-10), Preimmune sera (11).
Figure 3 illustrates oligosaccharide inhibition of the binding of L7-LOS to anti-L7-OH, de P-TT serum.
Figure 4 illustrates bactericidal activity of L7-OS-TT- and L7-OH, de P-TT antisera against *N. meningitidis* strain M982B.
Figure 5a illustrates the NMR spectrum of O-deacylated lipopolysaccharide (LPS-OH) derived from *N. meningitidis* L3 galE without gel column filtration showing broad unresolved lines in its NMR spectrum.
Figure 5b illustrates the NMR spectrum of O-deacylated lipopolysaccharide (LPS-OH) derived from *N. meningitidis* L3 galE after gel column filtration showing well resolved lines consistent with the structure of the LPS-OH material.
Figure 6 illustrates the CE-MS spectrum of dephosphorylated LPS (LPS-OH,deP) derived from *N. meningitidis* L3 galE.
Figure 7 shows the NMR spectra of a) LPS-OH and b) LPS-OH,deP of Figure 6.
Figure 8 shows the HPLC profile of LPS-OH,deP and CRM₁₉₇ over time (frames 1 to 3) and the HPLC profile of LPS-OH,deP linked through Kdo via a M₂C₂H linker.
Figure 9 illustrates that the inner core epitope was appropriately represented on the conjugate as evidenced by reactivity with Mab B5.
Figure 10 compares the immune response of MLC (lipid A route conjugates) with MLKC (Kdo route conjugates).
Figure 11 shows that the immune response to inner-core epitopes elicited by MLC-3 was bactericidal.
Figure 12 illustrates passive protection of MLC-3
Figure 13 shows ES-MS examination of LPS-OH of *Haemophilus influenzae* before (Fig.13a) and after (Fig.13b) alkaline phosphatase treatment and reveals peaks indicative of dephosphorylation of the LPS-OH.
Figure 14 shows CE-MS analysis on the doubly charged ions corresponding to the full and de-phosphorylated *Haemophilus influenzae* LPS-OH molecule confirming that the lipid A region of the molecule had lost a species of 80 amu consistent with loss of a phosphate molecule.
Figure 15 shows ¹H-NMR spectroscopy of a sample before and after dephosphorylation providing evidence for efficient and specific de-phosphorylation of the α-GlcN lipid A residue of *Haemophilus influenzae* (Fig.15a (phosphorylated) with Fig.15b (de-phosphorylated).
Figure 16 illustrates the inner-core LPS epitope of *Haemophilus influenzae* was appropriately presented on the conjugate as evidenced by reactivity with Mab LLA4.
Figure 17 shows a comparison of immune responses of SRA (lipid A route conjugates) (Fig.17b) derived sera to SK (Kdo route conjugates) (Fig.17a).
Figure 18 shows the immune response to *Haemophilus influenzae* conjugates using two different adjuvants (Ribi (R) and Freunds (F)). It was clear that the Immune response to these conjugates targeted inner-core epitopes in both whole LPS and LPS-OH molecules due to the broad cross-reactivity exhibited by the derived sera.

### DETAILED DESCRIPTION OF THE INVENTION

### Introduction to Examples 1 and 2

The invention of these two examples relates to meningococcal lipooligosaccharide-conjugate vaccines. The importance of appropriate presentation of conserved inner core oligosaccharide epitopes to the immune performance of meningococcal lipooligosaccharide-protein conjugate vaccines was demonstrated in the following experiments. Two different oligosaccharides were obtained by chemical degradations of the same L7 lipooligosaccharide and both were linked terminally to tetanus toxoid. One was a truncated oligosaccharide, in which the inner epitopes were incomplete and was obtained by mild acid hydrolysis of the L7 lipooligosaccharide. This oligosaccharide was conjugated by direct reductive amination through its newly exposed terminal KDO residue. The second, a full length oligosaccharide was obtained by O-deacylation of the L7 lipooligosaccharide, with subsequent removal of phosphate substituents from its lipid A moiety using alkaline phosphatase. This permitted the full length oligosaccharide, to be conjugated directly to tetanus toxoid by reductive amination through its newly exposed terminal 2-N-acyl-2-deoxy-glucopyranose (i.e., terminal glucosamine) residue. Comparison of the immune performance of the two conjugates in mice revealed, that while both were able to induce significant levels of L7-lipooligosaccharide-specific IgG antibody, only the conjugate made with the full length saccharide was able to induce antibodies which were bactericidal against homologous meningococci. Example 2 illustrates that similar results were obtained utilizing *Neisseria meningitidis* strain L3 *galE* conjugated to CRM₁₉₇, thus confirming the contributions of internal (i.e., inner- core) oligosaccharide epitopes to the induction of protective antibody. The LOS of *Neisseria meningitidis* strain L3 *galE* is comprised only of an inner- core oligosaccharide unit that does not contain the chain extension (i.e., lacto-N-neotetraose) observed in L7 LOS *Neisseria meningitidis* is a human pathogen of world-wide significance. Despite the success of current vaccines composed of groups A, C, W-135 and Y capsular polysaccharides, and the more recent improved group C polysaccharide conjugate vaccines (26), the group B polysaccharide is precluded from the above vaccines, even though it is a major contributor to the burden of disease in developed countries (23). This is because of the poor immunogenicity of the group B polysaccharide in both its native (33) and conjugated forms (4,12). Consequently alternative vaccines based on subcapsular antigens, including lipooligosaccharides are being explored.

The meningococcal LOS have been implicated in the immune response to natural infection (3,8), but their use as vaccines is contraindicated because of their high toxicity. They also exhibit considerable antigenic diversity, which also remains a major challenge. Currently there are 12 known different immunotypes (19,32,34,35), of which types L1-L7 are exclusively associated with groups B and C meningococci, and types L10-L12 with group A meningococci. Only types L8 and L9 overlap between the two groups. The epitopes responsible for the immunotyping are located in the oligosaccharide moieties of the LOS (13), which have been shown to be structurally diverse (5,7,14,16,21,22), but also to have some regions of similarity.

### Example 1 Production, characterisation and performance of a lipopolysaccharide-based meningococcal vaccine utilizing Neisseria meningitidis strain L7 conjugated to tetanus toxoid (TT) via alkaline phosphatase technology.

According to the invention, to avoid the toxicity of the LOS, the toxic lipid A moiety was removed by mild acid hydrolysis and subsequently the innocuous oligosaccharides were conjugated by different methods to protein carriers through their terminal 2-keto-3-deoxyoctulosonic acid (KDO) residues (9,13,30). Although L10-conjugates were able to induce in mice oligosaccharide antibodies which were bactericidal (9,13), conjugates made with oligosaccharides associated with groups B and C meningococci in comparison produced antisera with sub-optimal bactericidal activity (13,30). This was particularly noticeable in the case of the L3 and L7 immunotypes, which are unfortunately the most prevalent among groups B and C meningococcal isolates (32). Both the L3 and L7 immunotypes have similar structures; L7 being simply the desialylated form of L3 (22).

One possible reason for the sub-optimal immune performance of the above conjugates is that the point of cleavage of the LOS, at the KDO residue, is close to if not within the internal oligosaccharide epitopes, thus impairing their structures. The importance of internal epitopes to the immune response has been documented (15,24) and is due to structural similarity between the non-reducing distal oligosaccharide chain of the LOS and mammalian tissue antigens which results in immunodominance of the internal epitopes (14,15,20,29,31).

To test this hypothesis we compared the immune response of two L7 LOS -TT conjugates. Once made with terminally-linked hydrazine-treated full-length oligosaccharide (L7-OH, de P), and the other with a similarly linked but truncated oligosaccharide (L7-OS). Only the former was able to induce bactericidal antibodies in mice.

### MATERIALS AND METHODS

Strains M982B (serotype L7) and 406Y (serotype L3) were grown in Bacto Todd-Hewitt Broth (THB; Difco, Detroit, MI) at pH 7.3. Ten 5% chocolate agar plates (Quelab, Montreal, P.Q., (Canada), were inoculated with bacteria from frozen stock and incubated overnight at 37°C in an atmosphere of 5% CO₂. The bacteria were then resuspended in 50 ml of media (THB) and transferred to a screw-capped Erlenmyer flask containing 2 l of media (THB). The flask was shaken for 7 h at 37°C and the contents were transferred to a 25 l New Brunswick Scientific MFS-128S Microferm fermentor. The bacteria were grown, killed with 1 % formaldehyde, and harvested by centrifugation.

Isolation of LOS. LOS from the two serotypes were isolated by a previously described modified phenol-extraction procedure (7). They were finally purified by a fourfold ultracentrifugation for 6 h at 100,000 g using a Beckman LE-80 ultracentrifuge.

Analytical Methods. Solutions were evaporated at reduced pressure below 40°C in a rotary evaporator. Gel-filtration was carried out on columns (1.6 x 90 cm) of Bio-Gel P2 and P4 (Extra Fine, Bio-Rad Laboratories), using 0.02 M pyridine-acetate buffer (pH 5.4) as eluant and a flow rate of 12 ml/h. A Sephadex G-10 column (1.5 x 30 cm, Pharmacia) was also employed using water as eluant and a flow rate of 24 ml/h. Individual fractions were monitored using a Waters R403 differential refractometer.

Conjugates were analysed for their carbohydrate and protein contents using respective phenol-sulfuric acid (6) and bicinchoninic acid assays (27).

Nuclear Magnetic Resonance. NMR experiments were performed on a Bruker AMX, 500 spectrometer using a 5 mm broadband probe with ¹H coil nearest the sample. ¹H and ³¹P NMR spectra were recorded at 300 and 340 K in 5 mm tubes at concentrations of 3-5 µg of sample in 0.5 ml D₂O at pH 7.0 (¹H) and pH 7.6 (³¹P). Acetone was used as internal standard (2,225 ppm) for ¹H spectra. ³¹P NMR samples contained 5 mM EDTA and 2% DOC and were referenced to 85% orthophosphoric acid (0.00 ppm). All experiments were carried out with sample spinning. 2D hetero-correlated (HSQC) experiments were performed as previously described (2).

Chemical Methods. O-deacylation of the L3 and L7 LOS was performed using anhydrous hydrazine as previously described (22), to yield O-deacylated and partially dephosphorylated L3-OH and L7-OH. The core oligosaccharide (L7-OS) was obtained by heating the LOS (10 mg/ml) in 1% acetic acid at 100°C for 2 h. The insoluble lipid A was removed by centrifugation at 15,000 rpm for 15 min and the water soluble L7-OS was purified on a Bio-Gel P2 column. A similar procedure was used for the isolating of the L3-OS from its LOS except that the latter was hydrolyzed in 0.1 M acetate buffer at pH 4.2 for 2 h at 100°C.

Enzymatic Dephosphorylation of LOS-OH. LOS-OH (10 mg) were dissolved in 1 ml of 0.1 M ammonium bicarbonate (pH 8.0) and treated with 70 units of alkaline phosphatase (Boehringer Mannheim, Laval, P.Q., Canada) at 56°C for 18 h. At this time an additional 70 units of the same enzyme was added and the reaction was allowed to stand at 56°C for a further 6 h. The solution was then heated at 100°C for 5 min, centrifuged at 15,000 rpm for 5 min, and the partially dephosphorylated product (LOS-OH, de P) was purified on a Sephadex G-10 column.

Coupling of L7-OH, de P to Tetanus Toxoid. Using reductive amination procedures previously described (12,13), L7-OH, de P was conjugated to tetanus toxoid (TT). L7-OH, de P (10 mg) was dissolved in 200 µl of 0.02 M borate buffer at pH 9.0, together with TT (4 mg) and sodium cyanoborohydride (50 mg). The reaction was stirred for 4 d at 37°C and the progress of the conjugation was monitored by HPLC using a Superose 12 HR10/30 column (Pharmacia) with PBS as eluant. Conjugation was indicated by the gradual disappearance of the TT peak with the simultaneous appearance of the conjugate peak having a relatively lower Kₐᵥ value. When the TT peak had disappeared, the conjugate was purified on a Bio-Gel A 0.5 (Bio-Rad) column (1.6 x 42 cm) as was obtained in a yield of 5 mg. L7-OS was conjugated to TT using identical procedures (13).

Immunization Procedures. Groups of 6-8 week old 10 CF1 female mice (Charles River, St. Constant, Canada) were injected subcutaneously with each of the conjugates containing 2.5 µg of carbohydrate in saline solution. Together with RIBIs complete adjuvant (RIBI Immunochem Research Inc., Hamilton, MT) in a total volume of 0.2 ml. The mice were injected on day 0, 21, and 35 and the antisera were collected on day 45, filtered sterile and stored at -80°C.

ELISA. The wells of microtiter plates (Lynbro/Titertek, No. 76-381-04) were coated with solutions of LOS (2 µg/100 µg) in 0.05 M sodium carbohydrate buffer at pH 9.6 at 37°C for 3 h and then overnight at 4°C. The plates were then washed and blocked with 1% BSA in 20 mM. Tris-HC1-50mM NaC1 buffer containing 0.05% Tween 20 (T-TBS) pH 7.5 for 1 h at room temperature. The contents of the wells were then removed and serial dilutions (100 µl/well) of murine antisera in PBS were added and the plates were left for 2.5 h at room temperature. After washing with T-TBS, 100 µl of a 1:3000 dilution in PBS of an alkaline phosphatase-labelled goat anti-mouse IgG (H+L) (ICN. Aurora, OH), was added to each well. Following incubation for 1 h at room temperature the plates were washed with T-TBS (250 µl/well) and 100 µl/well of PNPP substrate (Kirkegaard and Perry Laboratories, Gaithersburg, MD) was added. The plates were allowed to stand for 1 h at room temperature and the optical densities were read at 410 nm using a Dynatech MR 5000 Microplate Reader. Isotyping experiments were performed using the same ELISA procedure as described above except with the substitution of peroxidase-labeled goat anti-mouse isotype-specific antibodies (Southern Biotechnology Associates Birmingham, AL) and the reading of the plates at 450 nm.

ELISA Inhibition. The wells of Lynbro/Titertek microtiter plates were coated with LOS, washed and blocked as described above. Concurrently a second microtiter plate containing serial two fold dilutions of inhibitors in PBS (50 µl total volume) was mixed with 50 µl of polyclonal antisera diluted 1:100 with PBS. The plate was incubated for 1.5 h at room temperature and the contents of the wells were transferred to the preprepared LOS-coated plate. The coated plate plus antibody and inhibitor was incubated for 2.5 h at room temperature and was processed and read as described in the ELISA-binding experiments above.

Bactericidal Assay. The bactericidal assays were carried out in tissue culture 96-well polystyrene plates (Costar, No. 3595) essentially as previously described (25). *N. meningitidis* strain M982B was grown overnight on chocolate agar plates (Quelab, Montreal, P.Q. Canada) at 37°C under a 5% CO₂ atmosphere, followed by inoculating a second plate and incubating it for 5 h. Two-fold dilutions of murine polyclonal antisera were made directly in the plate using Hanks' Balanced Salts (HBSS) containing 1% casein hydrolyzate, diluted to a final volume of 50 µl/well. A suspension of group B meningococci (GBM) in HBSS, 1% casein hydrolyzate was made giving an OD₄₉₀ = 0.29 and a final working dilution of bacteria was prepared by a further 1:20,000 dilution. Freshly thawed baby rabbit complement was added (20 µl) to each well, followed by 30 µl of the working dilution of bacteria (2,500 CFU/well). The plate was then shaken at 37°C for 1 h. The contents of each well were then mixed before plating (10 µl) on to chocolate agar. The agar plates were incubated overnight at 37°C, 5% CO₂ and the number of CFU were counted. The percent of killing was calculated relative to the mean values of either HBSS control wells or culture supernatant medium in the following manner: percentage of killing = (CFU_{control}-CFU_{mAb}/CFU_{control}) x 100.

### RESULTS

Characterization of Oligosaccharides. As previously described (13,22), application of a 1% acetic acid hydrolysate of the L7-LOS to a Bio-Gel P4 column yielded an oligosaccharide (L7-OS), whose structure was confirmed by ¹H NMR spectroscopy (22). Because it contained a terminal sialic acid residue, the L3-LOS was hydrolyzed under milder conditions (sodium acetate buffer at pH 4.2). The hydrolysis yielded two oligosaccharides which were separated by column chromatography (Bio-Gel P4). ¹H NMR spectra of the oligosaccharides indicated that one was the expected L3-OS (22) and the other its desialylated counterpart (L7-OS).

The L7-LOS was also O-deacylated using anhydrous hydrazine (22), prior to being further dephosphorylated with alkaline-phosphatase, to yield L7-OH, de P. In addition to O-deacylation, the structural changes wrought by each of the above procedures were monitored by ³¹P NMR spectroscopy, resulting in the determination of the structure of the oligosaccharide (L7-OH, de P) used for conjugation. The chemical shifts of the ³¹P NMR signals of the native and modified L7-LOS are listed in Table 1 and assignments were made largely by comparison with other ³¹P NMR studies on both LOS (22) and lipid A (1,11,17,18).

**TABLE 1. ³¹P NMR data obtained with L7 LOS and its modified products.**

| | | | Chemical Shift Values ^{a} (ppm) | | | | |
|---|---|---|---|---|---|---|---|
| L7 LOS and treatment with reagents | Pyrophosphorylethanolamine | | Pyrophosphomonoester | Phosphorylethanolamine | Monophosphate | | |
| | C-4' | C-1 | C-1,C-4 | C-3(Hep) | C-4' | C-1 | C-3' |
| L7 LOS | -9.53(1.0) | -10.36(1.4) | -5.21(0.3) | +0.70(1.0) | +5.21(0.3) | +2.77(0.2) | -^{b} |
| Hydrazine | -9.59(0.5) | -10.14(0.4) | - | +0.73(1.0) | +5.00(0.4) | - | - |
| Alkaline-phosphatase(1) | - | - | - | +0.76(1.0) | +5.21(0.5)^{d} | - | +3.4(0.3)^{c} |
| Alkaline-phosphatase(2) | - | - | - | +0.73(1.0) | +5.20(0.6) | - | +3.4(0.6)^{c} |

| | | | | | | | |
|---|---|---|---|---|---|---|---|
| ^{a} Values relative to the external standard H₃PO₄. ^{b} Not detected. ^{c} Tentative assignment. ^{d} Cross-peak with H-4' (HSQC experiment). | | | | | | | |

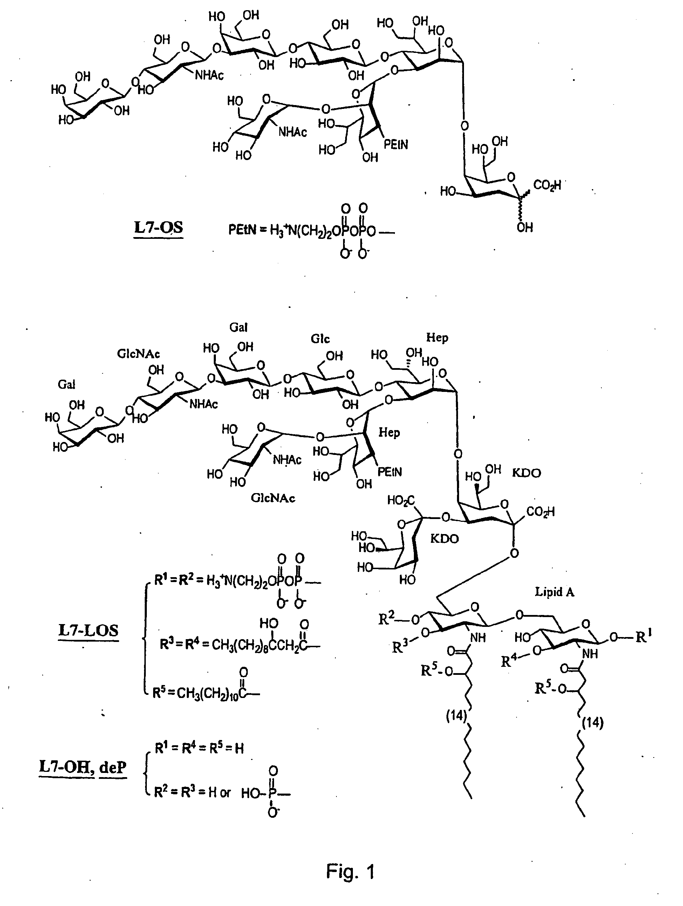

The native L7-LOS whose structure is depicted in Fig. 1 exhibited two diphosphorylethanolamine signals art-10.36 ppm and -9.53 ppm, which were assigned to C-1 and C-4 of the lipid A moiety (17). Another smaller signal at - 5.21 ppm was assigned to a pyrophosphomonoester group at C1 and/or C-4; of lipid A, which is consistent with previous assignments (17) on the meningococcal LOS, and with the conclusion that these positions are not all fully substituted with diphosphorylethanolamine groups (18). The signal at +0.70 ppm was assigned to a monophosphorylethanolamine substituent at 0-3 of one of the heptose residues (22). Treatment of the L7-LOS with hydrazine reduced by half the intensity of the signals at C-1 and C-4' and eliminated the pyrophosphomonoester signal at -5.21 ppm (18). It also produced two new signals at +4.97 ppm and +2.53 ppm which were assigned to monophosphate esters at C-4' and C-1 respectively. Thus hydrazine treatment is capable of partially degrading the dephosphorylethanolamine groups to monophosphate esters.

The remaining diphosphorylethanolamine groups at C-1 and C-4' were completely removed by the first alkaline phosphatase treatment, while the monophosphorylethanolamine phosphate substituent on the heptose residue remained intact. Some monophosphate ester remained on C-4', which was confirmed by a 2D hetero-correlated HSQC experiment, and some had migrated to C-3', but none remained on C-1. A second treatment with alkaline phosphatase failed to remove the residual monophosphate ester at C-4' and C-3', but however, the hemiacetal group of the terminal glucosamine residue of L7-OH, de P remained completely exposed and ready for conjugation.

Conjugation of Oligosaccharides to TT. Oligosaccharides L7-OS and L7-OH, de P were conjugated to TT using the procedure previously described by Jennings and Lugowski (12) but with minor modification. This time conjugations were carried out in 0.2 M borate buffer at pH 9.0, which improved the yields of the conjugates. Conjugation was easily monitored by HPLC because the conjugate peak was separated by 8 min from the TT peak, and was judged to be complete when the TT peak disappeared. The conjugates were purified by column chromatography and their stoichometric analysis is shown in Table 2. Both conjugates had comparable oligosaccharide/TT ratios.

**TABLE 2. Composition of L7-OS-TT and L7-OH, de P-TT conjugates.**

| Conjugate | % Saccharide^{a} | % TT^{b} | Molar ratio Saccharide/TT^{c} |
|---|---|---|---|
| LS-OS-TT | 20.0 | 79.0 | 18.9:1 |
| LS-OH, de P-TT | 26.2 | 72.5 | 15.5:1 |

| | | | |
|---|---|---|---|
| ^{a} Estimated by phenol-sulfuric assay using the appropriate saccharides as standards. ^{b} Protein content was estimated by the bicinchoninic acid assay using TT as the standard. ^{c} The molar ratio of saccharide/TT was calculated using molecular weights of 1590 for L7-OS, 2534 for L7-OH, de P and 150,000 for TT. | | | |

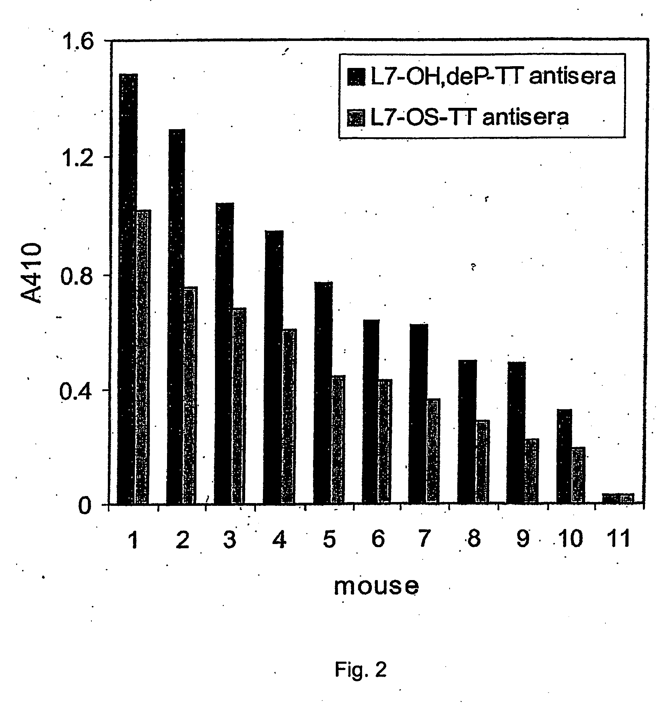

Immunogenicity of Conjugates in Mice. The L7-OS-TT and L7-OH, de P-TT conjugates were evaluated for their immunogenicity in mice. Each group of mice was given three subcutaneous injections with vaccines containing 2.5 µg of carbohydrate before being bled. The ELISA titers of the individual mice are shown in Fig. 2 using the L7-LOS as the coating antigen and show that the preimmune sera contained only trace amounts of L7-LOS-specific antibodies. While both conjugates induced antibody having this latter specificity, the levels induced by L7-OH, de P-TT were consistently higher than those induced by L7-OS-TT. In addition the subclass distribution of antibodies elicited by both conjugates was not significantly different (Table 3). Both conjugates were able to produce high levels of potentially bactericidal IgG2a, IgG2b and IgG3 antibodies.

**TABLE 3. Immunoglobulin isotype distribution of L7-OS-TT and L7-OH, de P-TT antisera.**

| Subclass | L7-OS-TT | L7-OH, de P-TT |
|---|---|---|
| IgG1 | 18.0% | 9.1% |
| IgG2a | 18.3% | 18.1% |
| IgG2b | 15.3% | 25.1% |
| IgG3 | 16.5% | 9.1% |
| IgM | 16.7% | 24.7% |
| IgA | 15.2% | 13.9% |

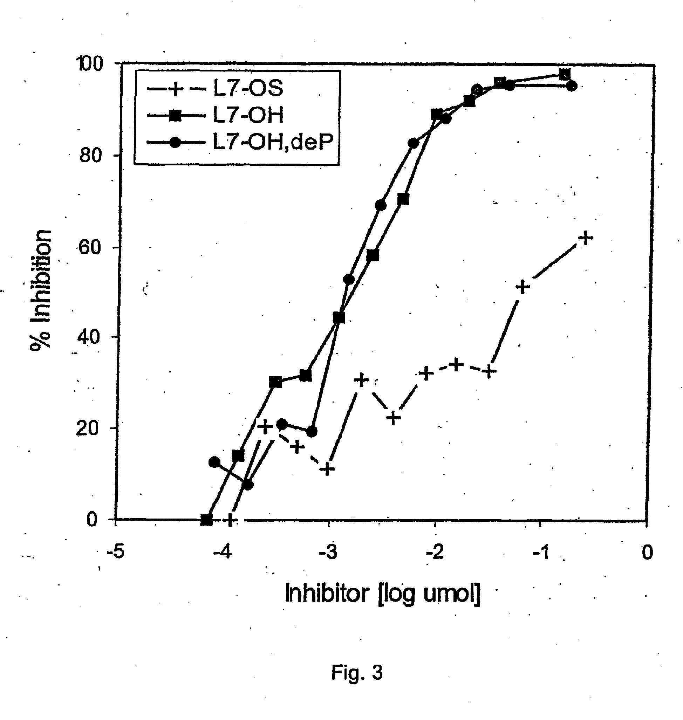

Inhibition of Polyclonal Antisera. To characterize the murine polyclonal antisera induced by the L7-OH, de P-TT conjugate a competitive inhibition ELISA was performed using L7-LOS as the coating antigen and six different oligosaccharides obtained from the L3-LOS and L7-LOS as inhibitors. The data compiled from the competitive inhibition ELISA experiments using the oligosaccharides obtained from the L7-LOS are shown in Fig. 3. Visual inspection of the curves obtained using the L7-LOS oligosaccharides reveal the most important feature, that unlike both L7-OH and L7-OH, de P, which were equivalent and good inhibitors of the above system, L7-OS was a very poor inhibitor.

The L3 LOS has the same structure as the L7-LOS except that it is terminally sialylated (22). Consistent with the inhibition results obtained using the oligosaccharides from the L7-LOS, within experimental error those obtained from the L3-LOS exhibited similar inhibition properties (data not shown). L3-OH and L3-OH, de P were good inhibitors of the binding of the polyclonal antiserum to the L7-OS. Whereas the L3-OS like that of the L7-OS was also a poor inhibitor of the system.

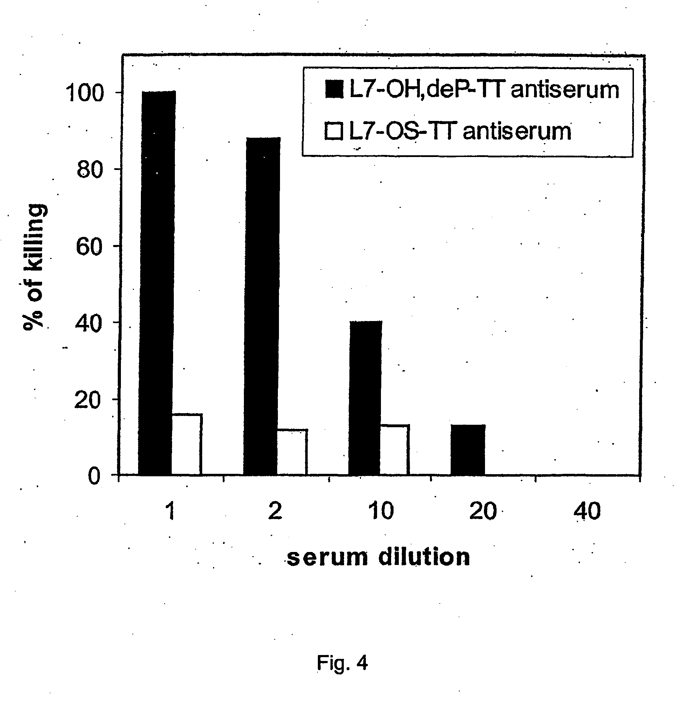

Bactericidal Activity of Antisera. The bactericidal activities of the antisera induced in mice by L7-OS-TT and L7-OH, de P-TT conjugates against the homologous immunotype organism are shown in Fig. 4. Although both conjugates were able to elicit L7-LOS-specific antibodies containing potential bactericidal isotypes, only the antisera induced by L7-OH, de P-TT was bactericidal to any degree. When diluted to 1:10 it still gave 40% killing, whereas even the undiluted antisera from the L7-OS-TT conjugate exhibited very poor bactericidal activity.

### DISCUSSION

In previous studies on LOS conjugate vaccines the problem of the toxicity of the LOS was avoided by removal of the toxic lipid A from the molecule by mild acid hydrolysis (9,13,20). Conjugates were then made using the non-toxic truncated core oligosaccharides as haptens. A number of different chemical procedures were used in the syntheses of the conjugates but all of them utilized the newly-exposed KDO residue as the linkage point (9,13,20). The conjugates were evaluated in animals and some general conclusions can be gleaned from the results obtained. Only conjugates made with immunotypes associated with group A strains (L8-L12) were able to induce antibodies having good bactericidal activity (9,13). Conjugates made with immunotypes associated with group B, and C strains, such as L3,7 induced antibody having no bactericidal activity (13,30). This was disappointing because L3,7 is the most prevalent immunotype found among group B *N. meningitidis* disease isolates (32). The ability of the group A associated immunotypes to induce bactericidal antibody is probably structure-based. Unlike the LOS of group B and C strains, which have an immunosuppressive lacto-N-neotetraose chain or its sialylated analog attached to a Hep in their inner structures (14,16,22), the LOS of group A strains have more immunogenic distal chain structures.

It is known that the L3 and L7 immunotypes are closely related differing only by the addition of terminal sialic acid to the lacto-N-neotetraose chain of the former (22). Therefore, in addition to the poor immunogenicity of the L3 and L7 immunotypes conferred on them by the above structural features, another factor to consider in their inability to raise bactericidal antibody, is that cleavage of the LOS at the internal KDO residue could result in structural impairment of the LOS inner epitope. To test this hypothesis we conjugated an L7 oligosaccharide, having conserved inner epitopes to TT, and compared the immune properties of the conjugate with that of a TT conjugate made as previously described with a truncated L7 core oligosaccharide. To achieve our objectives we chose to conjugate the hydrazine-treated detoxified L7 LOS to TT. This procedure was previously described for the synthesis of non-typeable *H. influenzae* LOS conjugates (10) in which they used internal KDO residues as the linkage point. However, these residues are close to, if not a part of the inner epitopes. Therefore, consistent with our original strategy of stringently attempting to ensure the conservation of internal epitopes, we linked the O-deacylated LOS through its terminal reducing glucosamine residue. By distancing the linkage from the region of potential internal epitopes we also hoped to avoid any possibility of linkage interference with this region. In order to conjugate L7-OH to TT by reductive amination we needed to remove all the phosphate substituents from the hemiacetal group at C-1 of the lipid A terminal glucosamine residue. This was achieved by further treatment of the detoxified L7-LOS with alkaline-phosphatase. Using a combination of hydrazide and alkaline-phosphatase treatments and following the procedures by ³¹P NMR spectroscopy, we were able to substantiate the complete removal of both diphosphorylethanolamine groups from C-1 and C-4' of the lipid A moiety. Although two monophosphate esters remained, one located at C-4', which probably could have been removed by a more judicious enzyme treatment, the hemiacetal at C-1, was found to be completely free.

Therefore the above oligosaccharide (L7-OH, de P) and L7-OS were conjugated to TT by direct reductive amination and both conjugates L7OH, de P-TT and L7OS-TT were shown by analysis to have similar saccharide/TT ratios. This, together with their structural similarities, enabled a legitimate comparison of their immune performances to be made. Both conjugates were able to induce antibodies that bound to the L7-LOS, although the conjugate containing the conserved epitope (L7OH, de P-TT) was clearly the superior immunogen. Confirmation that conservation of L7-LOS epitopes is a factor in obtaining an efficacious immune response was more dramatically demonstrated in comparing the bactericidal activities of the antibodies induced by both conjugates. The conjugate having conserved internal oligosaccharide epitopes (L7-OH, de P) induced antibodies that were bactericidal for homologous organisms, whereas antisera induced by the conjugate made with the truncated core oligosaccharide (L7-OS-TT) was not. This latter negative result is also consistent with previous studies using conjugates made with similar oligosaccharides of the L3 and L7 immunotypes (13,30).

The difference in the ability of the two conjugates to induce bactericidal antibodies is surprising, because both conjugates were able to produce L7-LOS-binding antibodies of isotypes, which at least had the potential of being bactericidal. Perhaps it is possible that the lack of bactericidal activity in the antibodies raised by L7-OS-TT could be explained by their low avidity, but we have good evidence to demonstrate that important bactericidal epitopes are lost when the L7-OS is subjected to mild acid hydrolysis.

This was determined in inhibition experiments in which the different oligosaccharides were used to inhibit the binding of the L7-LOS to antibodies induced by the L7-OH, de P-TT conjugate. Whereas both L7-OH and L7-OH, de P were equally good inhibitors of the binding, L7-OS was a very poor inhibitor of the same system. This result implies that both L7-OH, and L7-OH, de P contain epitopes not present in L7-OS and also that these epitopes are the source of a large proportion of the bactericidal antibodies. When the equivalent sialylated L3 oligosaccharides were substituted for those of L7 in the above inhibition experiments, they produced essentially similar inhibition curves, which is consistent with the structural evidence (22), that the L7-LOS is equivalent to the desialylated L3-LOS.

### Example 2: Production, characterisation and performance of a lipopolysaccharide-based meningococcal vaccine utilising Neisseria meningitidis strain L3 galE conjugated to CRM₁₉₇ via alkaline phosphatase technology.

### Materials and Methods

In this example, the galE mutant of Neisseria *meningitidis* strain H44/76 (L3 immunotype) was initially grown overnight at 37 °C in 10% CO₂ on 50% Todd-Hewitt 50% Columbia (THC) agar plates. Starter plates were used to heavily-inoculate starter cultures (1 L) and grown in (THC) broth at 37°C for 18 h. Starter cultures were used to inoculate the 28L fermenter with the same media, and grown as for starter cultures. After overnight growth (17h at 37°C), the culture was killed by addition of phenol (1 %), and chilled to 15°C and the bacteria were harvested by centrifugation (13, for 20min) (Wakarchuk, W., et al., 1996. J Biol. Chem. 271: 19166-19173). Generally yields were 100g biomass (wet wt.).

The crude LPS was extracted from the bacterial pellet using the standard hot phenol-water method (Westphal, O., and J. K. Jann. 1965. Meth. Carbohydr. Chem. 5: 83-91), treated with Dnase, Rnase and Proteinase K and purified from the aqueous phase by repeated ultracentrifugation (105,000 x G, 4°C, 2 x 5h) (Masoud, H., E. R. Moxon, A. Martin, D. Krajcarski, and J. C. Richards. 1997. Biochemistry 36: 2091-2103).

Preparation of O-deacylated LOS. LOS was dried overnight *in vacuo* and anhydrous hydrazine (98%; 1ml / 10 mg) LOS was added and the suspension stirred at 37°C for 30 - 45 min, cooled on ice and dry ice / acetone chilled (acetone (5 vols.) was added slowly). Solutions were centrifuged (10K, 30 min) and pellet was washed (x2) with acetone. The pellet was re-dissolved in H₂O and freeze dried to produce LOS-OH. Yield ∼ 50%.

LPS-OH was quality controlled by ES-MS in the negative ion mode on a VG Quattro (Fisons Instruments) or API 300 (Perkin-Elmer/ Sciex) triple quadrupole mass spectrometer. Samples were dissolved in water that was diluted by 50% with acetonitrile: water: methanol: 1% ammonia (4: 4: 1: 1) and the mixture was enhanced by direct infusion at 4µl / min. In some cases NMR (although addition of deuterated SDS and EDTA was usually needed to obtain a resolved spectrum) was utilized in a manner similar to that given in Example 1 (above).

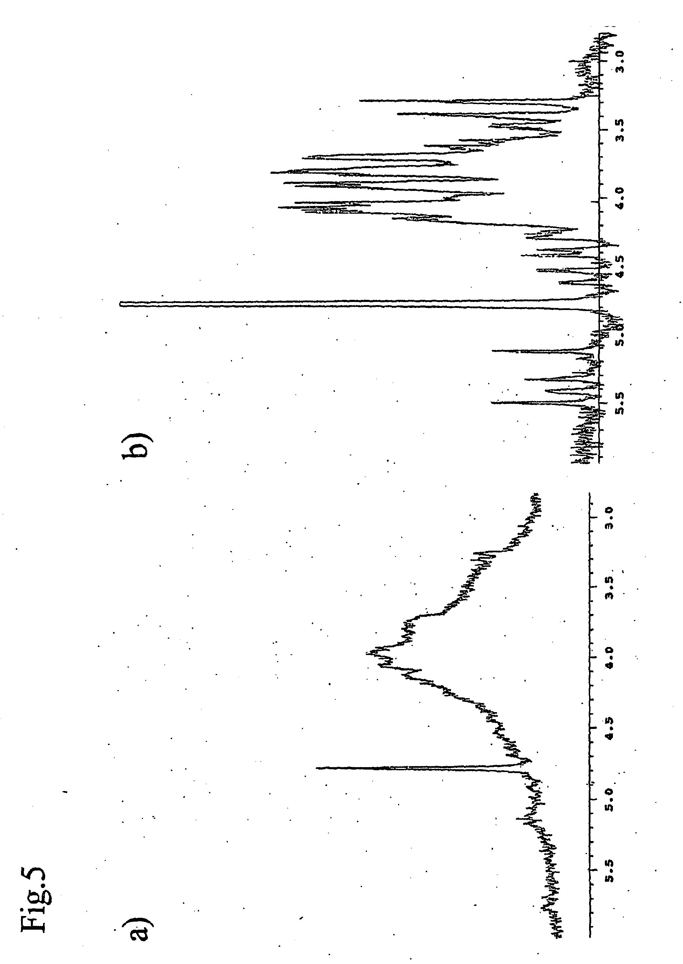

Purification of O-deacylated LPS to increase dephosphorylation efficiency. The L3 galE LOS-OH was dissolved in H₂O (∼10 mg / 4 - 5 ml). Any insoluble material was removed by centrifugation (14 K, 5 min). The resulting supernatant was applied to a 2.5 cm x 50 cm Sephadex G-50 (medium) gel filtration column and eluted with H₂O. The resulting eluant was monitored by refractive index and PhOH / H₂SO₄ carbohydrate assay (absorbance at 490ₙₘ). Carbohydrate-positive fractions were freeze dried individually and examined by ¹H-NMR spectroscopy after dissolving in 0.7 ml D₂O. Fractions producing well resolved NMR spectra were pooled and freeze dried. Yield - 50%. As (Fig.5b) illustrates NMR spectrum of G-50 purified material was consistent with the structure of the LPS-OH material. LPS-OH material that was not column purified in this way showed broad unresolved lines in its NMR spectrum (Fig.5a), indicating possible aggregation of material.

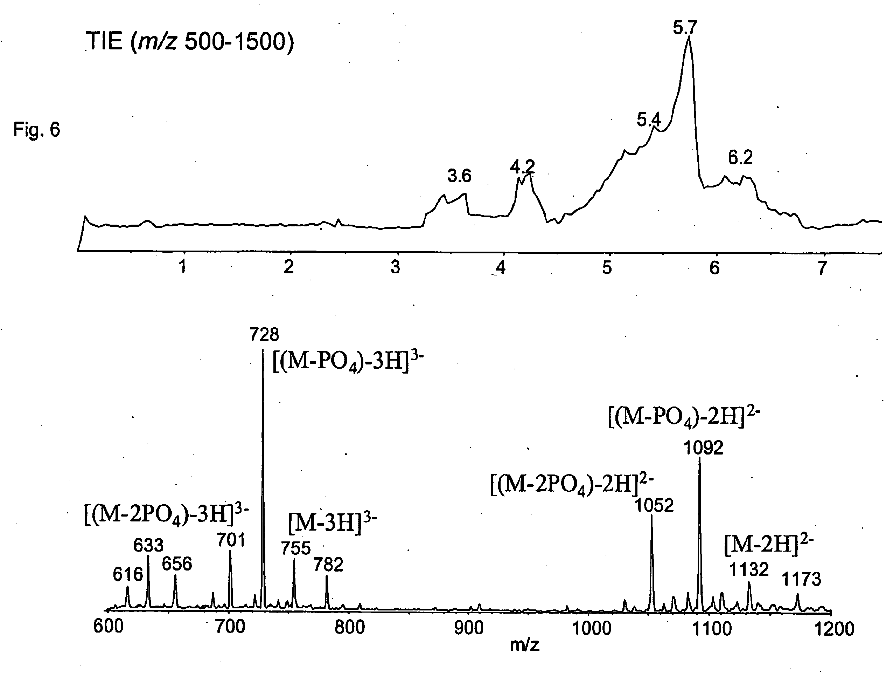

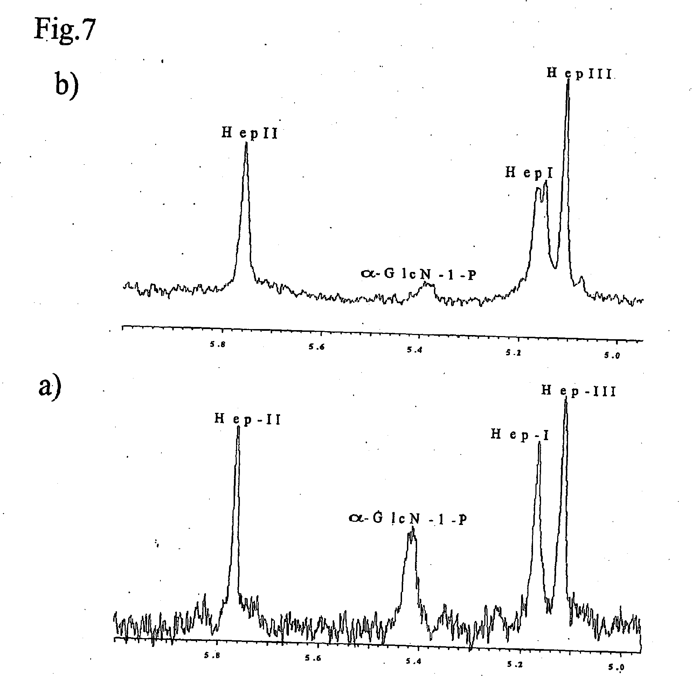

Alkaline phosphatase dephosphorylation of O-deacylated LPS (LPS-OH). The methods used were fundamentally those followed in Example 1 (above) with the following modifications as outlined below. The purified L3 *galE O*-deacylated LPS was treated with alkaline phosphatase (Sigma; P 6772, Type VII-L from bovine intestinal mucosa, affinity purified lyophilized powder in Tris-citrate buffer) as follows. The powder provided by the supplier was dissolved in 0.1 M NH₄HCO₃ buffer (pH 8.0) to give a 4,000 U / ml solution. A control reaction was always performed by dissolving ca. 1 mg of p-nitrophenylphosphate in 1 ml of 0.1 M NH₄HCO₃ buffer (pH 8.0). 20υl of enzyme was added and if the reaction proceeded, an immediate yellow colouration was observed. The purified *O-*deacylated LOS was dissolved in 0.1 M NH₄HCO₃ buffer (pH 8.0) at a concentration of 1 mg / ml and 100 U enzyme / mg of purified O-deacylated LOS was added. The reaction mixture was stirred in a reacti-vial at 54°C. After ∼ 16 h reaction an aliquot was removed, heated at 100°C for 5 min and cooled and centrifuged at 14 K for 5 min in an attempt to remove denatured enzyme. An aliquot of this was analysed by CE-MS (Fig.6), utilizing single ion monitoring to scan for diagnostic ions of the starting and dephosphorylated material. If successful de-phosphorylation was indicated by CE-MS, the remaining reaction mixture was denatured and centrifuged as above and lyophilised to reduce the amount of volatile buffer present. Freeze-dried material was dissolved in H₂O and de-salted on a Sephadex G-10 column, eluant monitored as above and carbohydrate positive fractions combined and freeze-dried. The yield of dephosphorylated reaction products was - 80-90%. A small amount of the dephosphorylated material was examined by ¹H-NMR spectroscopy after dissolving in 0.7 ml D₂O. Evidence for efficient de-phosphorylation was obtained by observation of loss of signal for the phosphorylated anomeric ¹H-resonance for the α-GlcN lipid A residue from 5.4 ppm (contrast Fig.7b with Fig.7a).

### Coupling of LPS-OH, de P to CRM₁₉₇ (see Scheme 1).

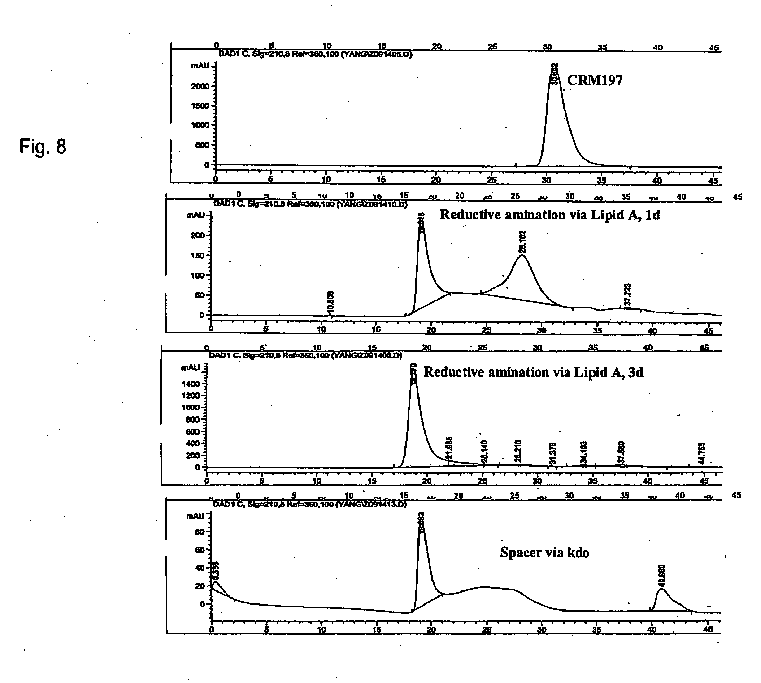

Coupling by reductive amination.—To a solution of CRM₁₉₇ (4.5 mg) in 0.1 N NaHCO₃ (0.15 mL) was added L3 *galE* LOS-OH, de P (2.0 mg) and NaCNBH₄ (5.0 mg). The mixture was kept at room temperature for two days. The progress of the conjugation was monitored by HPLC using a Superose 12 HR10/30 column (Pharmacia) with PBS as eluant at a flow rate of 0.4 mL/min (Fig.8). Conjugation was indicated by the complete disappearance of the CRM₁₉₇ peak and the simultaneous appearance of the conjugate peak with a relatively lower Kₐᵥ value. The mixture was repeatedly diluted with PBS (10 mL x 4) and concentrated by Centriprep (Amicon, 50,000 Dalton) at 3,000 r/min for 60 min to remove un-conjugated LPS. Final solution of conjugate (2.5 mL) was subjected to column purification (Biogel A 0.5, PBS) and the fractions were collected and concentrated by Centriprep. The LPS and CRM197 contents were analysed using the same methods described in *Example 1* and the ratio of LPS to CRM₁₉₇ was about 5.2:1.

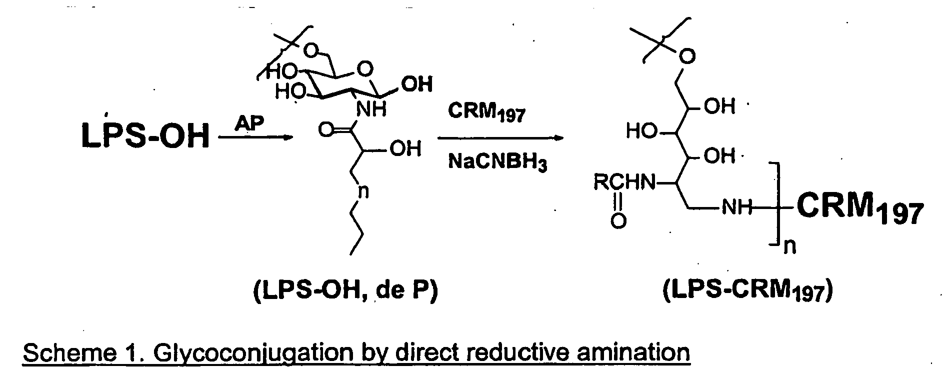

Conjugation or L3 *galE* LOS-OH by coupling through a Kdo carboxyl with a linker (M₂C₂H) (see Scheme 2) (modified procedure employed by Gu *et al* (op. cit.)).

Activation of Kdo with M₂C₂H.- To a solution of LPS-OH (2.0 mg) in water (1 mL) was added M₂C₂H (3 mg in 100 µL DMSO). The pH of the solution was adjusted to 4.7-4.8 using 0.01N NaOH or 0.01N HCl. EDC (3 mg in 100 µL water) was added and the pH of the solution was kept at 4.7-4.8 for 3 h. Purification was performed on a Sephadex G-10 column (40 x 1.6 cm) using water as eluant. Fractions containing activated LPS-OH were collected and lyophilized to amorphous (c.a. 2.0 mg) material. ¹H NMR (D₂O) shown a characteristic peak of meilaimide at 6.75 ppm.

Thiolated CRM₁₉₇. A solution of CRM₁₉₇ (6 mg) in 0.1N NaHCO₃ (0.2 mL) was diluted with a triethylamine buffer (pH 8.0). To this solution iminothiolane-HCl (0.5 mg) was added and the mixture was kept at room temperature for 2h. Small molecules were removed by Centriprep (10 mL x 3) until no thio compounds were detected in the washings.

Coupling.—To a solution of the above thiolated CRM₁₉₇ (0.8 mL) was added activated LPS-OH (2 mg) and the solution was kept at room temperature overnight. HPLC indicated successful conjugation (also see Fig.8). Dithioerythreitol (0.5 mg) was added and after 15 min purification was performed on Biogel A 0.5 column. Two fractions were collected and concentrated by Centriprep. The LPS and CRM₁₉₇ contents were analysed and the ratio of LPS to CRM₁₉₇ was about 6.5:1

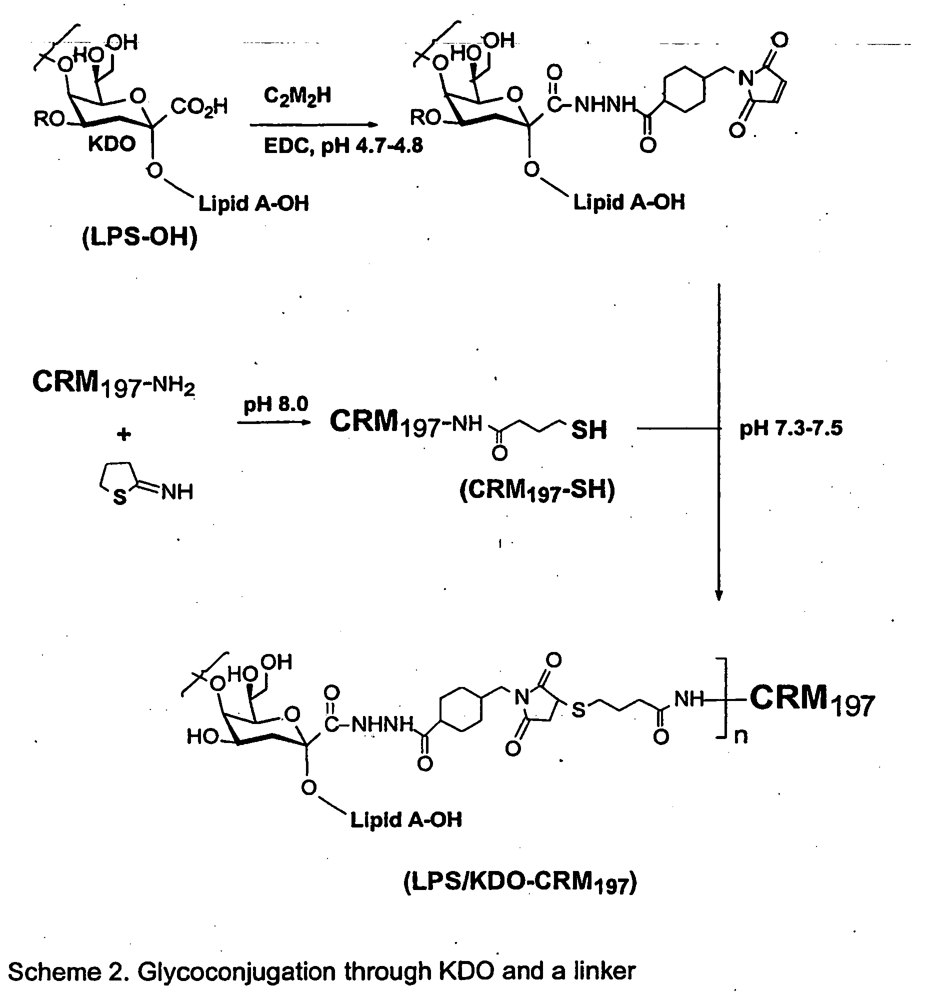

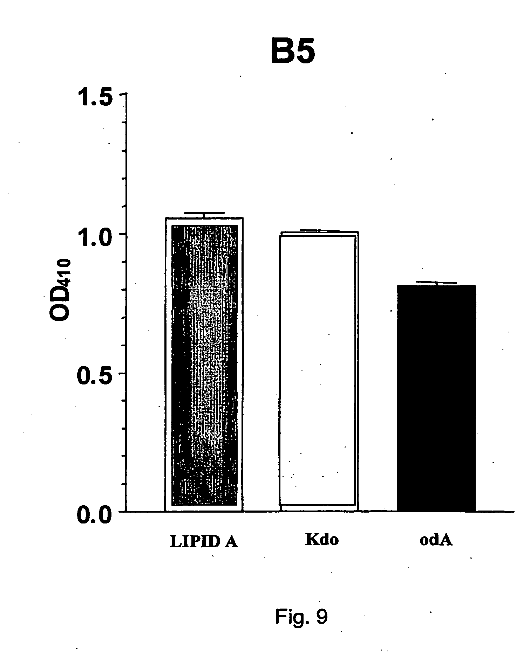

Confirmation of epitope presentation. The inner-core LPS epitope was appropriately presented on the conjugate as evidenced by reactivity with MAb B5 (Fig.9). It was previously shown that MAb B5 recognises a conserved inner-core epitope on meningococcal LOS in Plested et al 1999 reference #24 from reference list below).

Immunisation protocols. These were carried out as described in Plested et al 1999 with the following modifications. Briefly, female Balb/ C mice, 6-8 weeks of age were immunised intraperitoneally with L3 *galE* OdA LPS-CRM conjugates (MLC and MLKC). Each mouse received 2 µg of carbohydrate in 0. 2 ml Ribis complete adjuvant (Cedarlane Laboratories Ltd, Hornby, ON) per injection. The mice were boosted on day 20 and 42 with an equivalent amount of conjugated vaccine. Sera were recovered by terminal heart puncture on day 51.

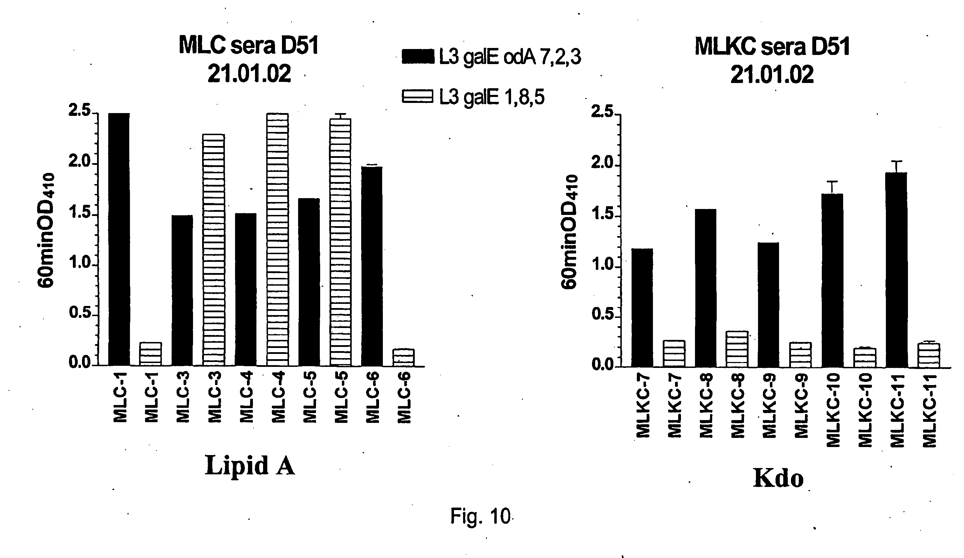

Comparison of immunological response to different conjugation strategies: Advantages of conjugation to carrier protein by coupling through the reducing terminus glucosamine. Comparison of immune responses of MLC (lipid A route conjugates) and MLKC (Kdo route conjugates) derived sera illustrated (Fig.10) that MLC derived sera generally recognise inner-core epitopes in the whole LPS background; whereas MLKC derived sera do not recognise inner-core epitopes in the LPS background, but the immune response targets the O-deacylated lipid A region of the molecule, which is not a desirable feature.

Serum Bactericidal (SB) assay. Serum bactericidal (SB) assay method was adapted from CDC protocol except polyclonal sera derived from mice following the conjugate immunizations was added to dilutions of human pooled sera and 1000 cfu of *Neisseria meningitidis* strain and incubation time was 40-45min at 37°C. Briefly, bacteria were grown up onto BHI agar overnight from frozen stocks. A suspension of bacteria in PBS-B was measured at OD260 (1: 50 in 1% SDS, 0.1 % NAOH). Using a 96-well microtitre plate 50µL buffer was added to wells in columns 2-7. 50µL of 80% decomplemented human pooled sera was added to column 8 wells. 50µL of 80% pooled sera was added to wells in column 1. Two-fold serial dilutions of antibody were added to columns 1-7 (discarding the last 50µL from column 7). 50µL of bacterial suspension diluted to give 1000 cfu in 50µL were added to wells of columns 1-8. The mixture was incubated for 40-45 minutes and plated out onto BHI agar for overnight incubation. The number of colonies on each plate was counted and the results expressed as a % of cfu/ml in decomplemented control well.

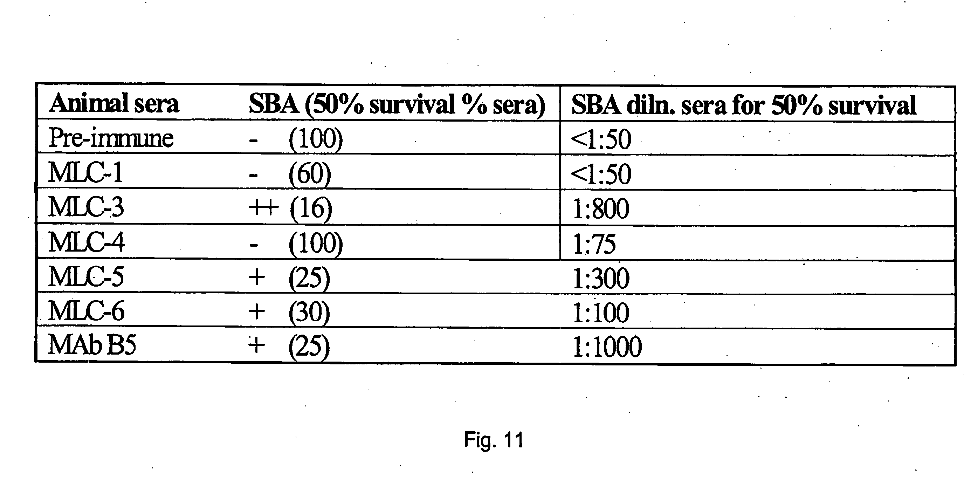

The SB assay illustrated that the immune response to the inner-core epitopes elicited by sera MLC-3 was bactericidal (Fig.11).

*In vivo* passive protection (PP) assay In vivo passive protection model using 5-day old Wistar infant rats model. This model was as described by Moe, G. R., et al., 1999. Infect. Immun. 67: 5664-5675, except higher doses of *Neisseria meningitidis* bacteria were used and different *Neisseria meningitidis* strain was used. Briefly, groups of 5 day old infant rats were randomized with mothers, weighed and given inoculum 1 X 10⁸ cfu/ml *Neisseria meningitidis galE* mutant mixed 1:1 with either (i) No antibody (PBS) (ii) irrelevant IgG₃(iii) pre-immune sera (iv and v) polyclonal sera MLC-3 and MLC-6 derived from mice following the conjugate immunizations (vi) Affinity purified MAb B5 (100 µg) (iv) MAb P1.2 (anti-porin antibody) (2 µg). Infant rats were monitored for signs of infection and sampled by tail vein bleed at 6 hours post-infection. Animals were weighed and terminal bleed was taken after 24h by cardiac puncture following injection of pentobarbitone. Neat and diluted blood were plated immediately onto BHI plates and incubated overnight. Plates were counted next day to determine bacteremia (cfu/ml) at 6h and 24h..

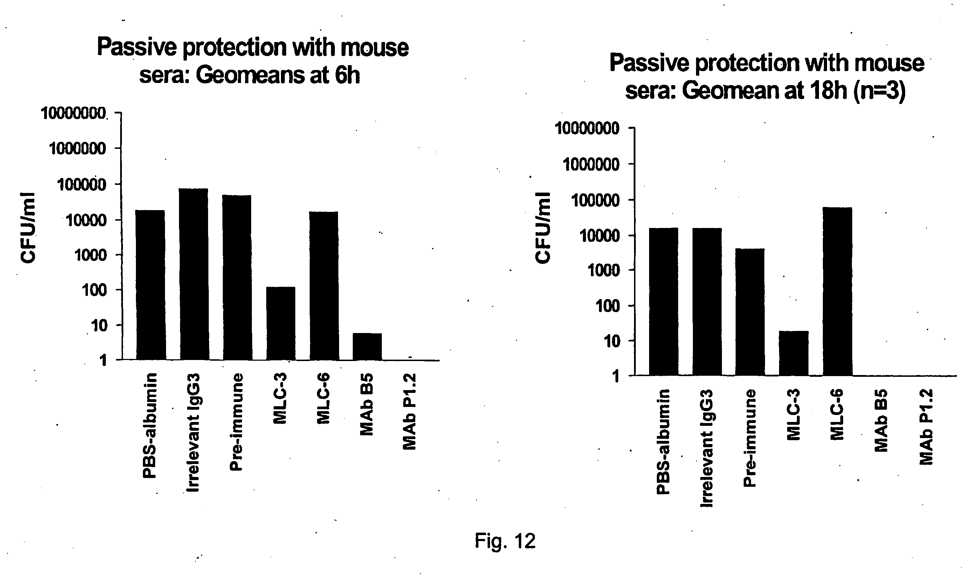

The PP assay illustrates that the immune response to the inner-core epitopes elicited by sera MLC-3 passively protected mice against challenge (Fig.12)

### Example 3: Production, characterisation and performance of a lipopolysaccharide-based Haemophilus influenzae vaccine utilising Haemophilus influenzae strain 1003 lic1 IpsA conjugated to TT via alkaline phosphatase technology.

### Introduction

*Haemophilus influenzae* is a major cause of disease worldwide. Six capsular serotypes ("a" to "f") and an indeterminate number of acapsular (non-typeable) strains of *H. influenzae* are recognized. Type b capsular strains are associated with invasive diseases, including meningitis and pneumonia, while non-typeable *H. influenzae* (NTHi) is a primary cause of otitis media in children and respiratory tract infections in adults. Otitis media is a common childhood disease which accounts for the highest frequency of paediatric visits in the United States (Stool et al., Pediatr. Infect. Dis. Suppl., 8:S11-S14, 1989).

The development of a vaccine for NTH*i* diseases has proved difficult because of a lack of understanding of the antigens that confer protective immunity. Efforts in developing a NTH*i* vaccine have been focused on cell surface antigens such as outer membrane proteins and pili or fimbria strains of *H*. *influenzae* (i.e. against disease caused by NTH*i*) because they are only protective against infections caused by *H. influenzae* strains bearing the type b capsule. Lipopolysaccharide (LPS) is a major-NTH*i* cell-surface antigen. LPS of *Haemophilus influenzae* has only been found to contain lipid A and oligosaccharide (OS) components. Because the lipid A component of LPS is toxic, it must be detoxified prior to conjugation to an immunogenic carrier, as discussed above. In this example a non-typable strain of *H*. *influenzae,* strain 1003 with specific mutations in the genes *lic1* and lpsA is utilized. As taught in WO02/16640, this double mutant strain of *H. influenzae* elaborates LPS that is comprised of the conserved *H. influenzae* inner-core oligosaccharide region attached to a lipid A moiety only.

### Materials and Methods

Unless stated otherwise, the methods used are described in Example 2. *H. influenzae* strain 1003 *lic1 IpsA* was grown at 37°C in brain heart infusion (BHI) broth supplemented with haemin (10µg/ml) and NAD (2µg/ml). For selection after transformation, kanamycin (10µg/ml) was added to the growth medium. The LOS was isolated by the phenol water extraction method, o-deacylated with anhydrous hydrazine and purified gel filtration chromatography on a Sephadex G-5o column as described above.

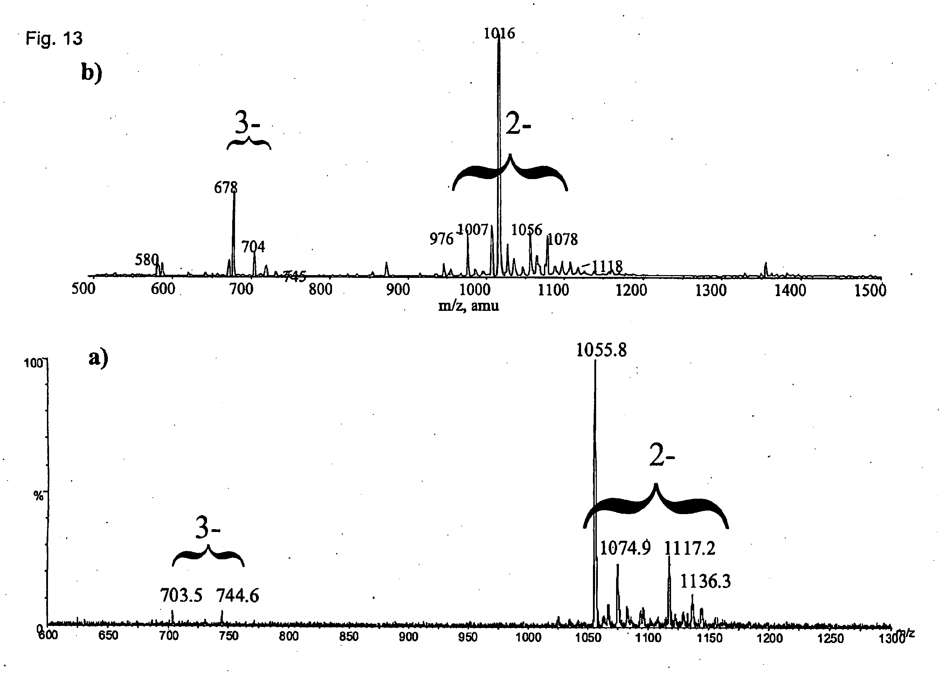

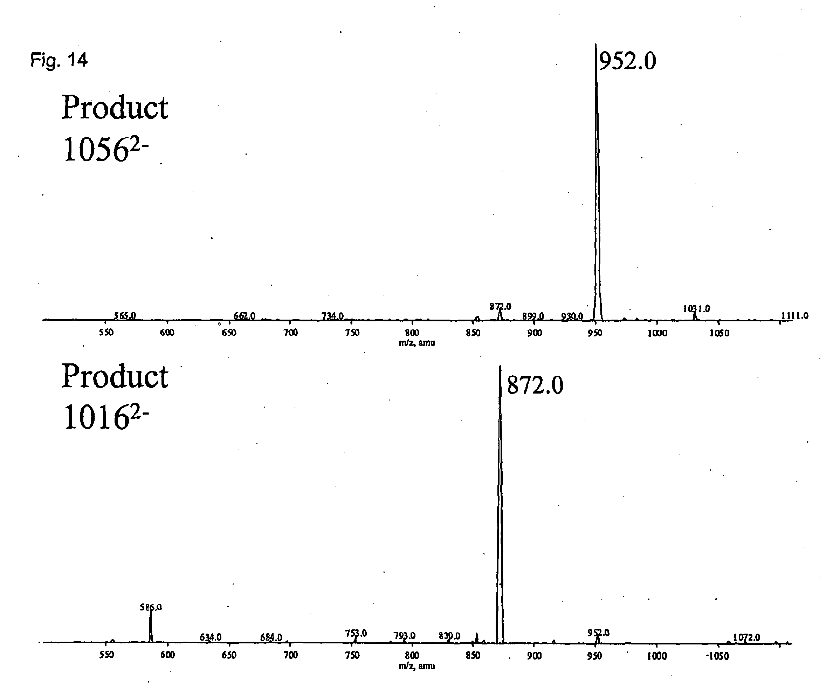

Enzymatic dephosphorylation of LOS-OH. Alkaline phosphatase treatment was essentially as described above. ES-MS examination of LPS-OH before (Fig.13a) and after (Fig.13b) alkaline phosphatase treatment revealed peaks indicative that dephosphorylation of the LPS-OH had taken place. Subsequent CE-MS analysis on the doubly charged ions corresponding to the full and dephosphorylated LPS-OH molecule confirmed that the lipid A region of the molecule had lost a species of 80 amu consistent with loss of a phosphate molecule (Fig.14).

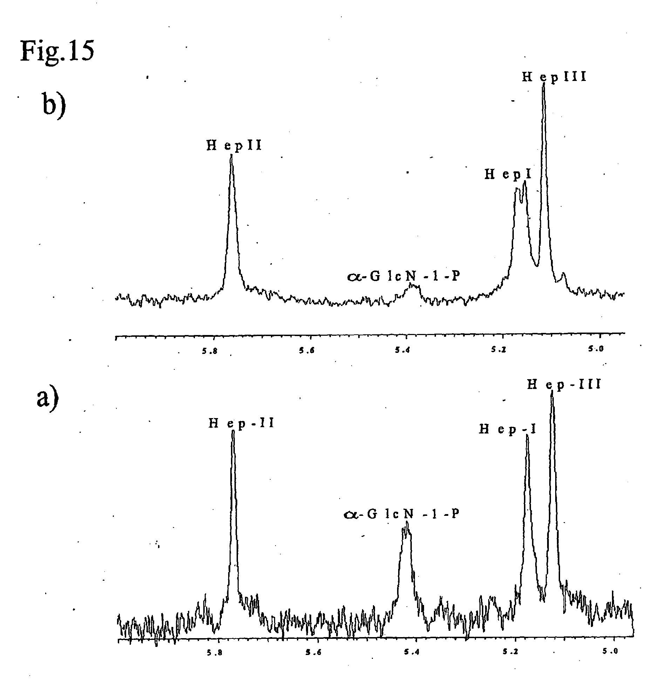

¹H-NMR spectroscopy of the sample before and after de-phosphorylation provided evidence for efficient and specific de-phosphorylation by observation of the loss of the signal for the phosphorylated anomeric ¹H-resonance for the reducing terminus α-GIcN of the O-deacylated LOS from 5.4 ppm (compare Fig.15a (phosphorylated) with Fig.15b (de-phosphorylated)).

Coupling of non-typable *Haemophilus Influenzae 1003lic1IpsA* LOS-OH, de-P to tetanus toxoid (see Scheme 3). In this example, a spacer is used to link the LPS-OH, de-P to TT as follows.

Activation of TT with Bromoacetyl groups (TTAcBr). -To a solution of 20 mg of TT (0.5 mL) in 1.6 mL of 0.1 M Na₂HPO₄ was added 20 mg of bromoacetyl succinyl ester (Sigma) in 0.4 ml of DMSO. The mixture was stirred at room temperature for 1 h when a TNBS test indicated almost complete derivatization of amino groups (TNBS test: the solutions of TT, TTAcBr (both at 3 mg/mL, 200 µL) and buffer were reacted with freshly prepared 5% TNBS in 0.1 M borate buffer (pH 8.3,400 µL) for 1.5 h. Only TT showed Yellow colour (420 nm)). The mixture was then passed through a Sephadex G-25 column (1.6 x 40 cm) using 10 mM phosphate buffer (pH 6.5) containing 1 mM EDTA as eluant. The first peak (protein) was collected (about 10 mL).

LPS Derivatization with a Spacer (LPS-OH, SS).-To a solution of LPS-OH, de P (10 mg) in 0.1 M NaHCO₃ (3 mL) were added cystamine (75 mg, Aldrich) and NaCNBH₃ (40 mg, re-crystallized to ensure purity). The mixture was kept at room temperature for 72h. The mixture was purified on a Sephadex G-25 column using water as eluant. The first peak was collected and lyophilized (10 mg). CE-MS analysis indicated that the conversion yield is about 50-60% based on the *m*/*z* of 1084 (product) and 1016 (starting material).

*Activation of LPS-OH, SS to LPS-OH, SH.-* Spacer derived LPS-OH, SS (10 mg) was dissolved in 2.0 mL of 0.1 M Na₂HPO₄ with 0.2 M DTT at RT for 1.5 h. The solution was passed through a Sephadex G-25 column (1.6 x 40 cm) using 10 mM phosphate buffer (pH 6.5) containing 1 mM EDTA as eluant. The first peak was collected (about 8 mL) and Ellman test showing strong positive (yellow colour) indicates the presence of thiol groups.

Coupling.-The solutions of activated TTAcBr and LPS-OH, SH were mixed and the pH was adjusted by the addition of 0.1 M Na₂HPO₄ to 7.5. The mixture (about 36 mL) was kept at room temperature for 30 h. The HPLC profile showed slight shift indicating the higher molecular weight conjugates are formed.

Removal of un-conjugated LPS. -The above reaction solution was concentrated to about 5 mL by centrifugation with Centriprep (Amicon, 50,000 Dalton) at 3,000 r/min for 60 min. The solution was diluted by the addition of PBS (10 mL, CMF, Sigma) and concentrated. The process was repeated six times when carbohydrate was not detected in last two washings. Each washing was tested by BCA assay and Phenol-sulphuric assay. Conjugates in PBS (36 mL) were obtained with TT at 1.5 mg/mL and LPS at 0.2 mg/mL. The ratio of LPS to TT was about 10:1.

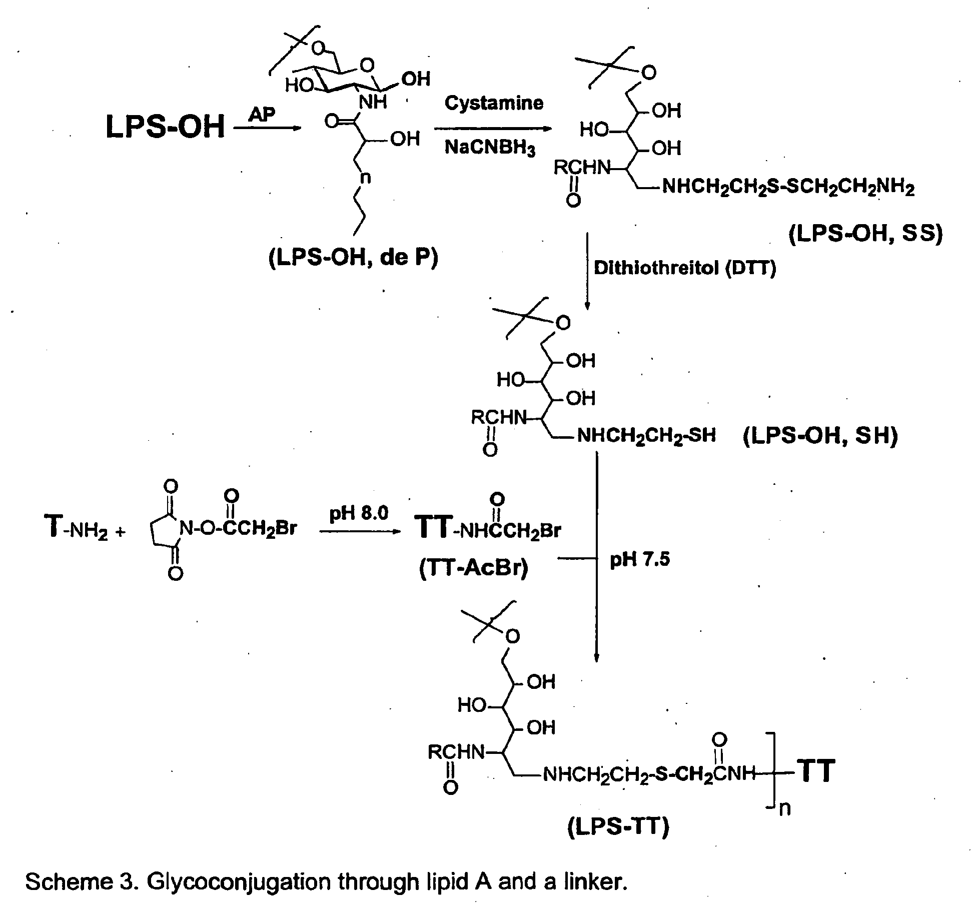

Coupling through Kdo and a linker. *Haemophilus influenzae* 1003 *lic1IpsA* LPS-OH was also coupled to TT via the Kdo group and a spacer by the methodology described in Example 2.

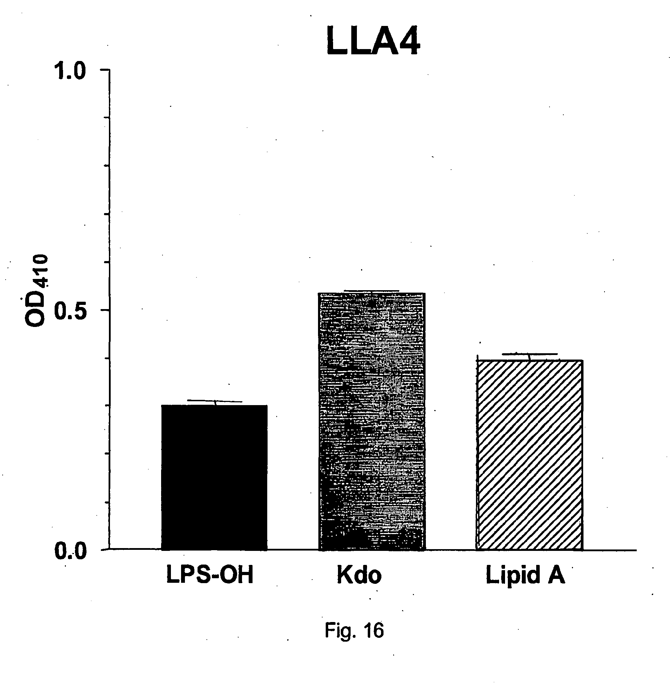

Confirmation of epitope presentation. The inner-core LPS epitope was appropriately presented on the conjugate as evidenced by reactivity with MAb LLA4 (Fig.16). It has been previously shown that MAb LLA4 recognises a conserved inner-core epitope on *Haemophilus influenzae* LPS (Example 6 in WO02/16640).

Immunization protocols These essentially followed those set of in Example 5 of WO02/16640 Female Balb/ C mice, 6-8 weeks of age were immunised intraperitoneally with 1003 *lic1, IpsA* OdA LPS-TT conjugates (SRA and SK). Each mouse received 10 µg of carbohydrate in 0. 2 ml Ribi or Freunds complete adjuvant per injection. The mice were boosted on day 21 and 42 with an equivalent amount of conjugated vaccine. Sera were recovered by terminal heart puncture on day 51.

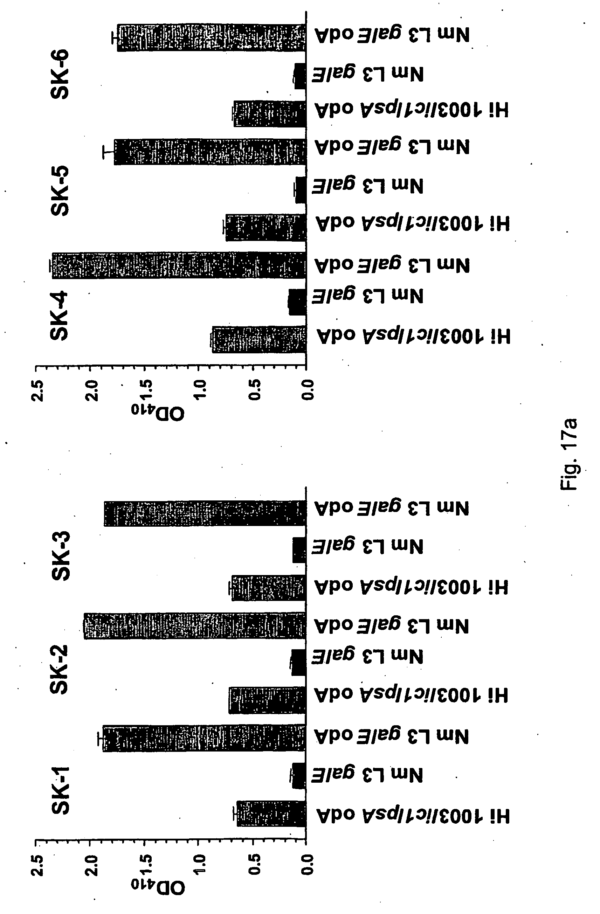

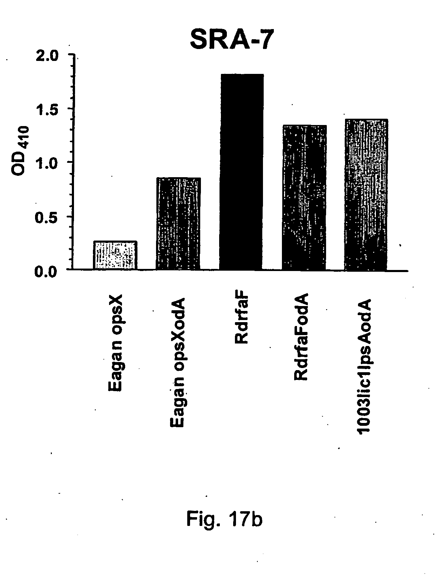

Comparison of immunological response to different conjugation strategies: Advantages of conjugation through the reducing terminus glucosamine-Comparison of immune responses of SRA (lipid A route conjugates with Ribi as the adjuvant) (Fig.17b) derived sera to SK (Kdo route conjugates) (Fig.17a) illustrated that SRA derived sera generally recognise inner-core epitopes in the whole LOS background; whereas SK derived sera significantly target the O-deacylated lipid A region of the molecule, which is not a desirable feature. Emphasising this behaviour was the observation that SK sera all recognised LOS-OH from the heterologous meningococcal strain L3 *gaIE* but did not recognize intact LOS from this L3 galE strain, confirming that a significant proportion of the immune response induced by immunisation with the 1003 *lic1, IpsA* OdA LPS-TT conjugates attached via the Kdo moiety, was directed to the O-deacylated lipid A region of the molecule.

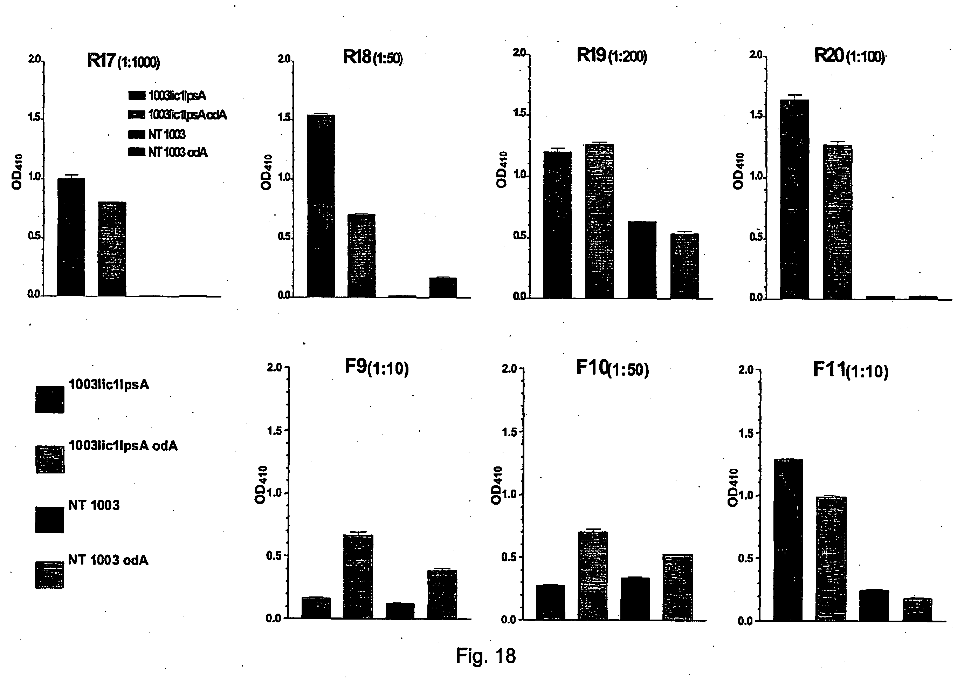

The studies that utilise the alkaline phosphatase technology to produce conjugates with the linkage to the reducing terminus glucosamine in the lipid A region of the carbohydrate moiety gave the following results. Using two different adjuvants (Ribi and Freunds) it was clear that the immune response to these conjugates targeted inner-core epitopes in both whole LPS and LPS-OH molecules. In Fig.18 R corresponds to Ribi adjuvant and F to Freunds adjuvant and odA refers to O-deacylated, LPS-OH molecules.

### Cross-reactivity of derived sera

Non-typable strains were obtained from Prof. Eskola as part of a Finnish Otitis Media Cohort Study and are mainly isolates obtained from the inner ear of children. These strains are further described in Hood et al., Mol. Microbiol. 33:679-792, 1999. 102 NTHi otitis media strains were sent to Richard Goldstein in Boston to be included in a survey of the diversity of over 600 *H. influenzae* capsulate and NTH*i* strains, obtained from around the world and over a 35 year period, by ribotyping analysis. When a dendrogram was drawn from the results of the ribotyping, the NTHi otitis media isolates were found to be present in almost all of the branches obtained. The 25 representative strains were selected from branches spanning the dendrogram and thus represent the known diversity associated with the species *H*. *influenzae.* Included in the 25 strains are some selected from the same cluster to allow an assessment of the diversity of closely related isolates.

Sera derived from immunisations with both Ribi and Freunds as adjuvants produced a broadly cross-reactive response against the LPS from the 25 non-typable strains of *Haemophilus influenzae* representative of the genetic diversity present in this species (Table 4).

**Table 4**

| 1003*lic1IlpsA*.TT D56 sera vs 25 NT LPS | | | | | | | | |
|---|---|---|---|---|---|---|---|---|
| strains (LPS) | F10 1:30 | F14 1:50 | F15 1:200 | F16. 1:100 | R19 1:200 | R20 1:100 | R22 1:200 | R24 1:30 |
| 1003 *lic1Ips* | 0.459 | 1.443 | 1.634 | 1.436 | 1.291 | 1.581 | 1.686 | 0.840 |
| •1268 | 0.848 | 0.132 | - | - | - | - | - | 1.098 |
| 1247 | 0.551 | 0.312 | - | - | 0.549 | - | - | 0.624 |
| •1209 | 0.443 | 0.212 | - | - | 0.473 | - | - | 0.556 |
| •1233 | 0.319 | 0.227 | - | - | 0.242 | - | - | 0.271 |
| •1181 | 0.522 | 0.283 | - | - | 0.489 | - | - | 0.755 |
| •1232 | 0.537 | 0.285 | - | - | 0.133 | - | - | 0.634 |
| 981 | 0.843 | 0.491 | - | - | - | - | - | 0.341 |
| 486 | 0.723 | 0.189 | - | - | - | - | 0.121 | 1.015 |
| •1292 | 0.555 | 0.358 | - | - | 0.468 | - | - | 0.541 |
| 1008 | 0.591 | 0.278 | - | - | 0.591 | - | - | 0.814 |
| 1158 | 0.624 | 0.267 | - | - | 0.163 | - | - | 0.640 |
| 1124 | 0.860 | 0.636 | - | - | - | - | - | 0.611 |
| 1003 batch II | 0.489 | 0.207 | - | - | 0.665 | - | - | 0.771 |
| •667 | 0.454 | 0.219 | - | - | 0.276 | - | - | 0.336 |
| •285 | 0.725 | 0.265 | - | - | 1.009 | - | - | 0.713 |
| •176 | 0.604 | 0.337 | - | - | 0.465 | - | 0.139 | 0.720 |
| •162 | 0.495 | 0.251 | - | - | 0.656 | - | - | 0.729 |
| •1180 | 0.549 | 0.193 | - | - | 0.652 | - | - | 0.784 |
| 1159 | 0.579 | 0.199 | - | - | 0.109 | - | - | 0.539 |
| 723+ NANA | 0.473 | 0.325 | - | - | 0.261 | - | - | 0.586 |
| •1231+ NANA | 0.335 | 0.397 | 0.126 | - | 0.123 | - | - | 0.471 |
| •1200 | 0.637 | 0.153 | - | - | - | - | - | 0.751 |
| •1207 | 0.370 | 0.156 | - | - | 0.571 | - | - | 0.593 |
| 432 | 0.330 | 0.196 | - | - | 0.276 | - | - | 0.500 |
| 375 + NANA | 0.306 | 0.194 | - | - | 0.330 | - | - | 0.407 |
| Rd opsX | 0.165 | 0.297 | - | - | - | - | - | - |
| Rd opsX odA | 0.810 | 0.566 | - | - | - | - | - | 0.822 |

| | | | | | | | | |
|---|---|---|---|---|---|---|---|---|
| Where F refers to sera produced using Freunds adjuvant and R refers to sera produced using Ribi Bold > 0.200 + > 0.100 | | | | | | | | |

### REFERENCES

1. Batley, M., N.H. Packer and J.W. Redmond. 1985. Analytical studies of lipopolysaccharide and its derivatives from Salmonella minnesota R595. Biochim. Biophys. Acta 821:179-194.
2. Baumann, H., A.O. Tzianabos, J.-R. Brisson, D.L. Kasper, and H.J. Jennings. 1992. Structural elucidation of two capsular polysaccharides from one strain of Bacteroides fragilis using high-resolution NMR spectrospcoy. Biochemsitry 31:4081-4089.
3. Brandtzaeg, P.P., P. Kierulf, P. Ganstäd, A. Skulberg, J.M. Bruun, S. Halvorsen, and E. Sorensen. 1989. Plasma endotoxin as a predictor of multiple organ failure and death in systemic meningococcal disease. J. Infect. Dis. 160:58-65.
4. Devi, S.J., J.B. Robbins, and R. Schneerson. 1991. Antibodies to poly [(2-8)-α-N-acetylneuraminic acid] and poly [(2-9)-α-N-acetylneuraminic acid] are elicited by immunization of mice with Escherichia coli K92 conjugates: potential vaccines for groups B and C meningococci and E. coli K1. Natl. Acad. Sci. U.S.A. 88:7175-7179.
5. DiFabio, J.L., F. Michon, J.-R. Brisson, and H.J. Jennings. 1990. Structure of the L1 and L6 oligosaccharide epitopes of Neisseria meningitidis. Can. J. Chem. 68:1029-1034.
6. Dubois, M., H. Gillis, J.K. Hamilton, A.A. Rebers, and R. Smith. 1956. Colorimetric methods for the determination of sugars and related substances. Anal. Biochem. 28:250-256.
7. Gamian, A., M. Beurret, F. Michon, J.-R. Brisson, and H.J. Jennings. 1992. Structure of the L2 lipopolysaccharide core oligosaccharides of Neisseria meningitidis. J. Biol. Chem. 267:922-925.
8. Goldschneider, I., E.C. Gotschlich and M.S. Artenstein. 1969. Human immunity to the meningococcus II. Development of natural immunity. J. Exp. Med. 129:1327-1348.
9. Gu, X.X., and C.-M. Tsai. 1993. Preparation characterization and immunogenicity of meningococcal lipopolysaccharide-derived oligosaccharide-protein conjugates. Infect. Immun. 61:1873-1880.
10. Gu, X.X., C.-M. Tsai, T. Veyama, S.J. Barenkamp, J.B. Robbins and D.J. Lim. 1996. Synthesis, characterization, and immunological properties of detoxified lipooligosaccharide from nontypeable Haemophilus influenzae conjugated to protein. Infect. Immun. 64:4047-4053.
11. Holst, O., S. Müller-Loennies, B. Lindner, and H. Brade. 1993. Chemical structure of the lipid A of Escherichia coli J-5. Eur. J. Biochem. 214:695-701.
12.Jennings, H.J. and C. Lugowski. 1981. Immunochemistry of groups A, B, and C meningococcal polysaccharide - tetanus toxoid conjugates. J. Immunol. 127:1011-1018.
13.Jennings, H.J., C. Lugowski and F.E. Ashton. 1984. Conjugation of meningococcal lipopolysaccharide R-type oligosaccharides to tetanus toxoid as a route to a potential vaccine against group B Neisseria meningitidis. Infect. Immun. 43:407-412.
14.Jennings, H.J., K.G. Johnson, and L. Kenne. 1983. The structure of an R-type oligosacchride core obtained from some lipopolysacchride of Neisseria meningitidis. Carbohydr. Res. 121:233-241.
15. Jennings, H.J., M. Beurret, A. Gamian, and F. Michon. 1987. Structure and immunochemistry of meningococcal lipopolysaccharides. Antonie van Leeuwenhoek 53:519-522.
16. Kogan, G., D. Uhrin, J.-R. Brisson and H.J. Jennings. 1997. Structural basis of the Neisseria meningitidis immunotypes including the L4 and L7 immunotypes. Carbohydr. Res. 298:191-199.
17.Kulshin, V.A., U. Zähringer, B. Lindner, C.E., Frasch, C.-M. Tsai, B.A. Dmitriev, and E.T. Rietschel. 1992. Structural characterization of the lipid A component of pathogenic Neisseria meningitidis. J. Bacteriol. 174: 1793-1800.
18. L'Vov, V.L., I.K. Verner, L.Y. Musina, A.V. Rodinov, A.V. Ignatenko and A.S. Shashkov. 1992. Structure of the sugar-phosphate moiety of lipid A from the lipopolysaccharide of Neisseria meningitidis group B, strain BC5S No. 125. Hydrolytic stability of phosphate and pyrophosphate substituents. Arch. Microbiol. 157:131-134.
19. Mandrell, R.E., and W.D. Zollinger. 1977. Lipopolysaccharide serotyping of Neisseria meningitidis by hemagglutination inhibition. Infect. Immun. 16:471-475.
20. Mandrell, R.E., J.M. Griffiss and B.A. Macher. 1988. Lipopolysaccharides (LOS) of Neisseria gonorrhoeae and Neisseria meningitidis have components that are immunochemically similar to precursors of human blood group antigens. J. Exp. Med. 168:107-726.
21. Michon, F., M. Beurret, A. Gamian, J.-R. Brisson and H.J. Jennings. 1990.. Structure of L5 lipopolysaccharide core oligosaccharides of Neisseria meningitidis. J. Biol. Chem. 265:7243-7247.
22. Pavliak, V., J.-R. Brisson, D. Uhrin, and H.J. Jennings. 1993. Structure of the sialylated L3 lipopolysaccharide of Neisseria meningitidis. J. Biol. Chem. 268:14146-14152.
23. Peltola, H., P.H. Mäkëla, and H. Kahty. 1977. Clinical efficacy of meningococcus group A polysaccharide vaccine in children three months to five years of age. N. Engl. J. Med. 297:686-691.
24. Plested, J.S., Makepeace, K., Jennings, M.P., Gidney, M.A.J., Lacelle, S., Brisson, J.-R., Cox. A.D., Martin, A., Bird, A.G., Tang, C.M., MacKinnon, F.M., Richards, J.C., and Moxon, E.R. 1999. Conservation and accessibility of an inner core lipopolysaccharide epitope of Neisseria meningitidis. Infect. Immun. 67, 5417-5426.
25. Pon, R.A., L. Lussier, Q.-L. Yang, and H.J. Jennings. 1997. N-propionylated group B meningococcal polysaccharide mimics a unique bactericidal capsular epitope in group B Neisseria meningitidis. J. Exp. Med. 185:1929-1938.
26. Richmond, P.R., R. Borrow, E., Miller, S. Clark, F. Sadler, A. Fox, N. Begg, R. Morris and K. Cartwright. 1999. Meningococcal serogroup C conjugate vaccine is immunogenic in infancy and primes for memory. J. Infect. Dis. 179:1569-1572.
27.Smith, P.K., R.I. Krohn, G.T. Hermanson, A.K. Mallia, F.H. Gartner, M.D. Provenzano, E.K. Fujimoto, N.M. Goeke, B.J. Olson, and D.C. Klenk. 1985. Measurement of protein using bicinchoninic acid. Anal. Biochem. 150:76-85.
28. Snippe, H. and J. Verhoef. 1991. Preparation characterization, and immunogenicity of meningococcal L2 and L3,9,7 phosphoethanolamine group containing oligosaccharide protein conjugates. Infect. Immun. 59:843-851.
29.Tsai, C.M., and C.V. Civin. 1991. Eight lipopolysaccharides of Neisseria meningitidis react with a monoclonal antibody which binds lacto-N-neotetraose. Infect. Immun. 59:1652-1656.
30.Verheul, A.F.M., A.K. Braaat, J.M. Leenhouts, P. Hoogerhout, J.T. Poolman, H. Snippe, and J. Verhoef. 1991. Preparation characterization, and immunogenicity of meningococcal L2 and L3,9,7 phosphoethanolamine group containing oligosaccharide protein conjugates. Infect. Immun. 59:843-851.
31.Verheul, A.F.M., G.J.P.H. Boons, A. Van der Marel, J.H. Van Boom, H.J. Jennings, H. Snippe, J. Verhoef, P. Hoogerhout, and J.T. Poolman. 1991. Minimal oligosaccharide structures required for induction of immune responses against meningococcal immunotype L1, L2, and L3, 9 phosphoethanolamine group-containing oligosaccharide-protein conjugates. Infect. Immun. 59:843-851.
32. Verheul, A.F.M., H. Snippe, H., and J.T. Poolman. 1993. Meningococcal lipopolysaccharide:virulence factor and potential vaccine candidate. Microbiol. Revs. 57:34-49.
33. Wyle, F.A., M.S. Artenstein, B.L. Brandt, D.L. Tramont, D.L. Kasper, P. Alteri, S.L. Berman and J.P. Lowenthal. 1972. Immunological response of man to group B meningococcal polysaccharide antigens. J. Infect. Dis. 126:514-522.
34. Zollinger, W.D., and R.E. Mandrell. 1977. Outermembrane protein and lipopolysaccharide serotyping of Neisseria meningitidis by inhibition of solid-phase radioimmunoassay. Infect. Immun. 28:451-458.
35.Zollinger, W.D., and R.E. Mandrell. 1980. Type-specific antigens of group A Neisseria meningtidis: lipopolysaccharide and heat-modifiable outer membrane proteins. Infect. Immun. 28:451-458.

## Claims

1. An antigenic detoxified reducing Gram-negative bacterial lipooligosaccharide having a detoxified lipid A region and linkable to an immunologically acceptable carrier through the detoxified lipid A region, said antigenic detoxified reducing bacterial lipooligosaccharide consisting of a moiety of the formula: 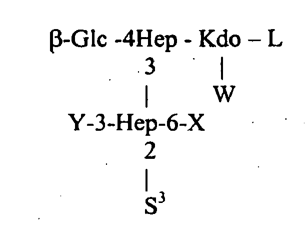
wherein L is the detoxified lipid A region;
Kdo is a 2-keto-3-deoxyoctulosonic acid moiety;
W is a 2-keto-3-deoxyoctulosonic acid moiety, a phosphate group, or a pyrophosphoethanolamine group;
Glc is glucose;
Hep is L-glycero-α-D-manno-heptose;
Y is phosphoethanolamine or hydrogen;
X is phosphoethanolamine when Y is hydrogen and X is hydrogen when Y is phosphoethanolamine; and
S³ is N-acetyl-α-glucosamine or L-glycero-α-D-manno-heptose.

2. The lipooligosaccharide of Claim 1 wherein S³ is N-acetyl-α-glucosamine.

3. The lipooligosaccharide of Claim 1 wherein W is a 2-keto-3-deoxyoctulosonic acid moiety and S³ is N-acetyl-α-glucosamine.

4. The lipooligosaccharide of Claim 1 wherein W is a phosphate group.

5. The lipooligosaccharide of Claim 1 wherein X is a phosphoethanolamine side moiety.

6. The lipooligosaccharide of Claim 1 wherein Y is a phosphoethanolamine side moiety.

7. A conjugate vaccine for combating a Gram-negative bacterium comprising an antigenic, detoxified reducing bacterial lipooligosaccharide according to any one of Claims 1 to 6 linked to an immunologically acceptable carrier through the detoxofied lipid A region.

8. The conjugate vaccine of Claim 7 wherein said antigenic, detoxified reducing bacterial lipooligosaccharide is a *Neisseria meningitidis* antigenic, detoxified reducing bacterial lipooligosaccharide.

9. The conjugate vaccine of Claim 7 wherein said antigenic, detoxified reducing bacterial lipooligosaccharide is a *Haemophilus influenzae* antigenic, detoxified reducing bacterial lipooligosaccharide.

10. The conjugate vaccine of any one of Claims 7 to 9, wherein said immunogenic carrier is a protein.

11. The conjugate vaccine of Claim 10, wherein said immunogenic carrier protein is selected from the group consisting of tetanus toxin/toxoid, cross-reacting material (CRM), NTHi high molecular weight protein, diphtheria toxin/toxoid, detoxified P. aeruginosa toxin A, cholera toxin/toxoid, pertussis toxin/toxoid, Clostridium perfringens exotoxins/toxoid, hepatitis B surface antigen, hepatitis B core antigen, rotavirus VP 7 protein, respiratory syncytial virus F and G proteins.

12. The conjugate vaccine of Claim 11, wherein said immunogenic carrier protein is tetanus toxoid.

13. The conjugate vaccine of Claim 11, wherein said immunogenic carrier protein is CRM₁₉₇.

14. A conjugate vaccine for combating a Gram-negative bacterium comprising an antigenic, detoxified reducing bacterial lipooligosaccharide according to any one of Claims 1 to 6 linked via a linker to an immunologically acceptable carrier through the detoxified lipid A region.

15. The conjugate vaccine of Claim 14, wherein said linker is selected from the group consisting of M₂C₂H, cystamine, adipic acid di-hydrazide, ε-aminohexanoic acid, chlorohexanol dimethyl acetal, D-glucuronolactone and p-nitrophenylethyl amine.

16. The conjugate vaccine of Claim 15, wherein said linker is M₂C₂H.

17. The conjugate vaccine of Claim 15, wherein said linker is cystamine.

18. A pharmaceutical composition comprising the conjugate vaccine of any one of Claims 7 to 17 in association with an adjuvant.

19. The pharmaceutical composition of Claim 18, wherein said adjuvant is selected from the group consisting of Freund's adjuvant, alum and Ribi.

20. The pharmaceutical composition of Claim 18 or 19 further comprising a pharmaceutically acceptable diluent.

21. Use of an antigenic, detoxified reducing bacterial lipooligosaccharide according to any one of Claims 1 to 6 in manufacture of a medicament for combating a Gram-negative infection in a mammal.

## Patentansprüche

1. Antigenes, entgiftetes, reduzierendes, gramnegatives, bakterielles Lipooligosaccharid, das einen entgifteten Lipid-A-Bereich hat und durch den entgifteten Lipid-A-Bereich an einen immunologisch zulässigen Träger koppelbar ist, wobei das antigene, entgiftete, reduzierende, bakterielle Lipooligosaccharid aus einem Molekülteil der Formel 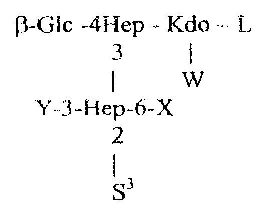 besteht, in der
L der entgiftete Lipid-A-Bereich ist,
Kdo ein 2-Keto-3-deoxyoctulosonsäureteil ist,
W ein 2-Keto-3-deoxyoctulosonsäurerest, eine Phosphatgruppe oder eine Pyrophosphoethanolamingruppe ist,
Glc Glucose ist,
Hep L-Glycero-α-D-manno-heptose ist,
Y Phosphoethanolamin oder Wasserstoff ist,
X Phosphoethanolamin ist, wenn Y Wasserstoff ist, und X Wasserstoff ist, wenn Y Phosphoethanolamin ist, und
S³ *N*-Acetyl-α-glucosamin oder L-Glycero-α-D-manno-heptose ist.

2. Lipooligosaccharid des Anspruchs 1, bei dem S³ N-Acetyl-α-glucosamin ist.

3. Lipooligosaccharid des Anspruchs 1, bei dem W ein 2-Keto-3-deoxy-octulosonsäureteil ist und S³ N-Acetyl-α-glucosamin ist.

4. Lipooligosaccharid des Anspruchs 1, bei dem W eine Phosphatgruppe ist.

5. Lipooligosaccharid des Anspruchs 1, bei dem X ein Phosphoethanolamin-Seitenteil ist.

6. Lipooligosaccharid des Anspruchs 1, bei dem Y ein Phosphoethanolamin-Seitenteil ist.

7. Konjugat-Impfstoff zur Bekämpfung eines gramnegativen Bakteriums mit einem antigenen, entgifteten, reduzierenden, bakteriellen Lipooligosaccharid nach einem der Ansprüche 1 bis 6, das durch den entgifteten Lipid-A-Bereich an einen immunologisch annehmbaren Träger gekoppelt ist.

8. Konjugat-Impfstoff des Anspruchs 7, bei dem das antigene, entgiftete, reduzierende, bakterielle Lipooligosaccharid ein für Neisseria meningitis antigenes, entgiftetes, reduzierendes, bakterielles Lipooligosaccharid ist.

9. Konjugat-Impfstoff des Anspruchs 7, bei dem das antigene, entgiftete, reduzierende, bakterielle Lipooligosaccharid ein für Haemophilus influenzae antigenes, entgiftetes reduzierendes bakterielles Lipooligosaccharid ist.

10. Konjugat-Impfstoff eines der Ansprüche 7 bis 9, bei dem der immunogene Träger ein Protein ist.

11. Konjugat-Impfstoff des Anspruchs 10, bei dem das immunogene Trägerprotein aus der Gruppe ausgewählt ist, die aus Tetanustoxin/toxoid, kreuzreagierendem Material (CRM), hochmolekularem Protein NTHi, Diphtherietoxin/toxoid, entgifteten P. aeruginosa Toxin A, Choleratoxin/toxoid, Keuchhustentoxin/toxoid, Exotoxine/toxoid des Clostridium perfringens, Hepatitis-Virus-B-Antigen, Hepatitis-B-Kern-Antigen, Rotavirus-VP 7-Protein, Proteine des Atmungssynzytialvirus F und G besteht.

12. Konjugat-Impfstoff des Anspruchs 11, bei dem das immunogene Trägerprotein Tetanustoxoid ist.

13. Konjugat-Impfstoff des Anspruchs 11, bei dem das immunogene Trägerprotein CRM₁₉₇ ist.

14. Konjugat-Impfstoff zur Bekämpfung eines gramnegativen Bakteriums mit einem antigenen, entgifteten, reduzierenden bakteriellen Lipooligosaccharid nach einem der Ansprüche 1 bis 6, das durch den entgifteten Lipid-A-Bereich über einen Linker an einen immunologisch annehmbaren Träger gekoppelt ist.

15. Konjugat Impfstoff des Anspruchs 14, bei dem der Linker aus der Gruppe ausgewählt ist, die aus M₂C₂H, Cystamin, Adipinsäuredihydrazid, ε-Aminohexansäure, Chlorhexanoldimethylacetal, D-Glucuronolacton und p-Nitrophenylethylamin besteht.

16. Konjugat.-Impfstoff des Anspruchs 15, bei dem der Linker M₂C₂H ist.

17. Konjugat-Impfstoff des Anspruchs 15, bei dem der Linker Cystamin ist.

18. Pharmazeutische Zusammensetzung mit dem Konjugat-Impfstoff eines der Ansprüche 7 bis 17 in Verbindung mit einem Adjuvans.

19. Pharmazeutische Zusammensetzung des Anspruchs 18, bei der das Adjuvans aus der Gruppe ausgewählt ist, die aus Freundschem Adjuvans, Aluminiumverbindung und Ribi besteht.

20. Pharmazeutische Zusammensetzung des Anspruchs 18 oder 19, ferne mit einem pharmazeutisch zulässigen Verdünnungsmittel.

21. Verwerdung eines antigenen, entgifteten, reduzierenden, bakteriellen Lipooligosaccharids nach einem der Ansprüche 1 bis 6 zur Herstellung eines Heilmittels zur Bekämpfung einer gramnegativen Infektion in einem Säuger.

## Revendications

1. Lipooligosaccharide bactérien Gram-négatif réducteur détoxifié antigénique ayant une région de lipide A détoxifiée et pouvant être lié à un vecteur immunologiquement acceptable via la région de lipide A détoxifiée, ledit lipooligosaccharide bactérien réducteur détoxifié antigénique consistant en un groupement de formule : 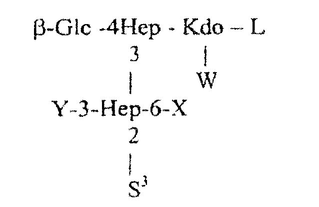
où L est la région de lipide A détoxifée ;
Kdo est un groupement acide 2-céto-3-désoxyoctulosonique ;
W est un groupement acide 2-céto-3-désoxyoctulosonique, un groupe phosphate ou un groupe pyrophosphoéthanolamine ;
Glc est le glucose ;
Hep est le L-glycéro-α-D-manno-heptose ;
Y est la phosphoéthanolamine ou l'hydrogène ;
X est la phosphoéthanolamine quand Y est l'hydrogène et X est l'hydrogène quand Y est la phosphoéthanolamine ; et
S³ est la N-acétyl-α-glucosamine ou le L-glycéro-α-D-manno-heptose.

2. Lipooligosaccharide selon la revendication 1 où S³ est la N-acétyl-α-glucosamine.

3. Lipooligosaccharide selon la revendication 1 où W est un groupement acide 2-céto-3-désoxyoctulosonique et S³ est la N-acétyl-α-glucosamine.

4. Lipooligosaccharide selon la revendication 1 où W est un groupe phosphate.

5. Lipooligosaccharide selon la revendication 1 où X est un groupement latéral de phosphoéthanolamine.

6. Lipooligosaccharide selon la revendication 1 où Y est un groupement latéral de phosphoéthanolamine.

7. Vaccin conjugué pour combattre une bactérie Gram-négative comprenant un lipooligosaccharide bactérien réducteur détoxifié antigénique selon l'une quelconque des revendications 1 à 6 lié à un vecteur immunologiquement acceptable via la région de lipide A détoxifiée.

8. Vaccin conjugué selon la revendication 7 où ledit lipooligosaccharide bactérien réducteur détoxifié antigénique est un lipooligosaccharide bactérien réducteur détoxifié antigénique de *Neisseria meningitidis.*

9. Vaccin conjugué selon la revendication 7 où ledit lipooligosaccharide bactérien réducteur détoxifié antigénique est un lipooligosaccharide bactérien réducteur détoxifié antigénique de *Haemophilus influenzae.*

10. Vaccin conjugué selon l'une quelconque des revendications 7 à 9 où ledit vecteur immunogène est une protéine.

11. Vaccin conjugué selon la revendication 10 où ladite protéine vecteur immunogène est choisie dans le groupe consistant en la toxine/anatoxine tétanique, une substance à réaction croisée (CRM), la protéine de haute masse moléculaire NTHi, la toxine/anatoxine diphtérique, la toxine A de P. aeruginosa détoxifiée, la toxine/anatoxine cholérique, la toxine/anatoxine coquelucheuse, les exotoxines/anatoxine de Clostridium perfringens, l'antigène de surface de l'hépatite B, l'antigène de nucléocapside de l'hépatite B, la protéine VP 7 de rotavirus, les protéines F et G de virus syncytial respiratoire.

12. Vaccin conjugué selon la revendication 11 où ladite protéine vecteur immunogène est l'anatoxine tétanique.

13. Vaccin conjugué selon la revendication 11 où ladite protéine vecteur immunogène est CRM₁₉₇.

14. Vaccin conjugué pour combattre une bactérie Gram-négative comprenant un lipooligosaccharide bactérien réducteur détoxifié antigénique selon l'une quelconque des revendications 1 à 6 lié via un lieur à un vecteur immunologiquement acceptable via la région de lipide A détoxifiée.

15. Vaccin conjugué selon la revendication 14 où ledit lieur est choisi dans le groupe consistant en M₂C₂H, la cystamine, le di-hydrazide d'acide adipique, l'acide ε-aminohexanoïque, le diméthylacétal de chlorohexanol, la D-glucuronolactone et la p-nitrophényléthylamine.

16. Vaccin conjugué selon la revendication 15 où ledit lieur est M₂C₂H.

17. Vaccin conjugué selon la revendication 15 où ledit lieur est la cystamine.

18. Composition pharmaceutique comprenant le vaccin conjugué selon l'une quelconque des revendications 7 à 17 en association avec un adjuvant.

19. Composition pharmaceutique selon la revendication 18 où ledit adjuvant est choisi dans le groupe consistant en l'adjuvant de Freund, l'alun et Ribi.

20. Composition pharmaceutique selon la revendication 18 ou 19 comprenant en outre un diluant pharmaceutiquement acceptable.

21. Utilisation d'un lipooligosaccharide bactérien réducteur détoxifié antigénique selon l'une quelconque des revendications 1 à 6 dans la production d'un médicament pour combattre une infection Gram-négative chez un mammifère.
